# EUROPEAN PATENT APPLICATION

(11) **EP 4 667 497 A1**
(43) Date of publication of application: **24.12.2025**
(21) Application number: 24756360.4
(22) Date of filing: 18.02.2024
(51) Int. Cl.: C07K 16/46, C07K 19/00

(54) **BISPECIFIC ANTIBODY AND USE THEREOF**

(30) Priority: 13.02.2023 CN 202310106092
(71) Applicant: Shaoxing Institute, Zhejiang University, Shaoxing, Zhejiang 312099 (CN)
(72) Inventor: HUANG, Deli, Shaoxing, Zhejiang 312099 (CN); CHEN, Dingrui, Shaoxing, Zhejiang 312099 (CN)
(74) Representative: Elzaburu S.L.P.
(86) International application number: PCT/CN2024/077450
(87) International publication number: WO 2024/169990

(57) **Abstract**

Provided is a bispecific antigen-binding molecule or a fragment thereof, comprising a first antigen-binding domain and a second antigen-binding domain, wherein the first antigen-binding domain is used for specifically binding to a cell surface antigen, and the second antigen-binding domain is used for specifically binding to vesicular stomatitis virus glycoprotein (VSV-G). The bispecific antigen-binding molecule or the fragment thereof can efficiently mediate VSV-G lentivirus infection of cells that do not express VSV-G receptor LDLR.

## Description

### CROSS REFERENCE TO THE RELATED APPLICATIONS

The present application claims the priority benefit of Chinese Patent Application No. 2023101060920 filed on February 13, 2023, which is incorporated herein by reference in its entirety.

### TECHNICAL FIELD

The present disclosure belongs to the field of immunology, and particularly relates to a bispecific antibody and use thereof.

### BACKGROUND TECHNOLOGY

In recent years, gene therapy has entered a golden age of rapid growth. Gene therapy aims to cure diseases or enhance disease resistance by replacing defective genes or adding new genes, hopefully treating a range of diseases such as cancer, cystic fibrosis, heart disease, diabetes, hemophilia and AIDS. As the application of delivery systems in gene therapy gradually matures, the barriers of the medicine transformation in gene therapy have been gradually broken through. The most common delivery systems are viral vectors, as viruses can enter cells by specifically recognizing cell surface receptors and deliver their carried genetic materials into target cells. In order to overcome potential risks, researchers continue to select and improve viral vectors to ensure patients' safety and maximize delivery efficiency. To date, the U.S. Food and Drug Administration (FDA) has approved eight gene therapy methods using three different types of viral vectors, which are adeno-associated virus (AAV) vectors, lentiviral vectors and herpes simplex viral vectors. As of February 2022, 25 types of treatment protocols using viral vectors have been in late-stage development, with additional 120 in Phase II trials. Among all three types of viral vectors, lentiviral vectors rank second in utilization, which have been used in 30% of gene therapies, second only to adeno-associated virus vectors.

As a member of the Retroviridae family, lentiviruses possess several characteristics that make them suitable for gene therapy: they can carry exogenous genes up to 10 kb; they can integrate genes into the genome of target cells for stable expression, and can infect both dividing and non-dividing cells; and they induce a low immune response in target cells.

The main steps of lentiviral infection of host cells include binding to the host cells, fusing with the host cell membrane to release structural proteins, enzymatic proteins and viral cores, reverse transcription of viral RNA by reverse transcriptase to form a pre-integration complex with integrase, catalysis of integration of the pre-integration complex into the host genome by integrase after the pre-integration complex entering into the nucleus, and expression in the cytoplasm of the exogenous gene driven by the upstream promoter thereof. Among these, the interaction between the glycoproteins on viral surface and the receptor proteins on host cell surface can be used to achieve the infection of specific cells. The first and most widely used glycoprotein is vesicular stomatitis virus glycoprotein (VSV-G), due to its broad tropism, high lentiviral titer produced and high stability.

The low-density lipoprotein receptors (LDL-Rs) are the main entry receptors for VSV-G pseudotyped lentiviruses on the cell surface. The LDL-R is a triacontahexahedral single-chain glycoprotein composed of 839 amino acid residues which contains 5 functional domains. It is widely distributed on the cell membrane surface of various tissues throughout the body including hepatocytes, vascular smooth muscle cells, monocytes and macrophages. The extracellular domain of LDL-R includes a ligand-binding domain, an epidermal growth factor precursor homology domain and a C-terminal domain rich in O-linked oligosaccharides, wherein the ligand-binding domain consists of 7 repeat sequences rich in cysteine (CR1-CR7). VSV-G can independently bind to two different CR domains (CR2 and CR3). Although LDLR is expressed in almost all tissues, its expression is extremely low on the surface of resting T cells, B cells or hematopoietic stem cells (HSCs), resulting in very low infection efficiency of these cells. The modification and utilization of these cells via lentiviral methods have been greatly limited, either in scientific experiments or in clinical applications such as CAR-T cell immunotherapy.

### CONTENT OF THE INVENTION

In order to solve this issue, the present disclosure provides a new technology that uses a bispecific molecule to mediate a gene delivery independent of the VSV-G glycoprotein receptor LDLR.

In one aspect, the present disclosure provides a bispecific antigen-binding molecule or a fragment thereof, comprising a first antigen-binding domain and a second antigen-binding domain, wherein the first antigen-binding domain is for specifically binding to a cell surface antigen; the second antigen-binding domain is for specifically binding to a vesicular stomatitis virus glycoprotein (VSV-G)

In another aspect, the present disclosure provides a bispecific antigen-binding molecule or a fragment thereof, comprising:
a first polypeptide comprising a VH, a CH1, a CH2, a CH3, a CR2 and a CR3; and a second polypeptide comprising a VL and a CL;
wherein VH is the heavy chain variable region of the first antigen-binding domain, VL is the light chain variable region of the first antigen-binding domain, the first antigen-binding domain is used for specifically binding to a cell surface antigen; CH1, CH2 and CH3 are respectively the first, second, and third constant regions of an IgG molecule, CL is the light chain constant region; CR2 and CR3 are the second and third CR domains of a low-density lipoprotein receptor (LDL-R).

In another aspect, the present disclosure also provides a bispecific antigen-binding molecule or a fragment thereof, comprising:
a first antigen-binding domain comprising an scFv, the scFv comprises a VH and a VL; and a second antigen-binding domain comprising a CR2 and a CR3; and optionally, CH2 and CH3 domains for linking the scFv and the second antigen-binding domain;
wherein the first antigen-binding domain is used for specifically binding to a cell surface antigen; VH is the heavy chain variable region, VL is the light chain variable region; CH2 and CH3 are respectively the second and third constant regions of an IgG molecule; CR2 and CR3 are the second and third CR domains of a low-density lipoprotein receptor (LDL-R).

In another aspect, the present disclosure also provides a bispecific antigen-binding molecule or a fragment thereof, comprising:
a first antigen-binding domain comprising a VHH; and a second antigen-binding domain comprising a CR2 and a CR3; and optionally, CH2 and CH3 domains for linking the VHH and the second antigen-binding domain;
wherein the first antigen-binding domain is used for specifically binding to a cell surface antigen; CH2 and CH3 are respectively the second and third constant regions of an IgG molecule; CR2 and CR3 are the second and third CR domains of a low-density lipoprotein receptor (LDL-R).

In another aspect, the present disclosure also provides a bispecific antigen-binding molecule or a fragment thereof, comprising:
a first antigen-binding domain comprising a natural ligand of a cell surface protein; and a second antigen-binding domain comprising a CR2 and a CR3; and optionally, CH2 and CH3 domains for linking the natural ligand and the second antigen-binding domain;
wherein the first antigen-binding domain is used for specifically binding to a cell surface antigen; CH2 and CH3 are respectively the second and third constant regions of an IgG molecule; CR2 and CR3 are the second and third CR domains of a low-density lipoprotein receptor (LDL-R).

In another aspect, the present disclosure also provides a polynucleotide encoding the bispecific antigen-binding molecule or the fragment thereof provided by the present disclosure.

In another aspect, the present disclosure also provides a recombinant vector comprising the polynucleotide provided by the present disclosure.

In another aspect, the present disclosure also provides a host cell comprising the polynucleotide or the recombinant vector provided by the present disclosure.

In another aspect, the present disclosure also provides a method for producing the bispecific antigen-binding molecule or the fragment thereof of the present disclosure.

In another aspect, the present disclosure also provides a method for transducing a target gene into a subject using a lentiviral vector, comprising administering the bispecific antigen-binding molecule or the fragment thereof provided by the present disclosure to the subject.

Beneficial effects of the present disclosure:
(1) The present disclosure designs a brand-new bispecific molecule, which can efficiently mediate the VSV-G lentiviral infection of cells not expressing the VSV-G receptor LDLR. In the present disclosure, the antigen-binding domain for specifically binding to a cell surface antigen can be widely replaced to achieve specific targeting of different types of cells.
(2) The inventors have also found that for some cells that are difficult to be infected (with low or no LDLR expression), such as primary cells, etc., the use of bispecific molecules can greatly improve the transduction efficiency of the target gene, thereby significantly increasing the probability of integrating the target gene into the host cell genome, which provides a favorable way for constructing stable cell lines and exploring gene functions such as overexpression, knockdown or knock-down of specific genes.
(3) Due to the numerous advantages of lentiviral vectors, they have become the primary vectors for infecting T cells, especially in the production of CAR-T cells. However, on the one hand, viral production is difficult to scale up; on the other hand, the limited number of T cells and the restriction of activation efficiency further increase the difficulty in the production of CAR-T cells. The bispecific molecule of the present disclosure enhances the infection efficiency of lentiviruses on T cells, and can reduce the requirements for the number of T cells and viral titer when preparing CAR-T cells, thereby further lowering the production costs of CAR-T cells.

### DESCRIPTION OF THE DRAWINGS

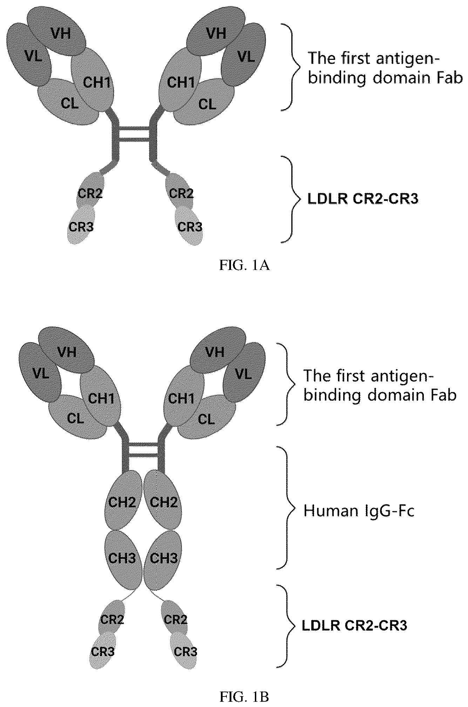

FIGS. 1A to 1F show schematic diagrams of the structures of the bispecific antigen-binding molecules of the present disclosure; wherein the end for binding to cells can be designed in the form of Fab, scFv, single-domain antibody VHH or a natural ligand of specific membrane proteins on the cell surface; the end for binding to VSV-G pseudotyped lentiviruses is the CR2 and CR3 domains of the human low-density lipoprotein receptor (LDLR), which can also be replaced with the CR2 and CR3 domains of LDLRs from pig, horse or cattle species; the two binding domains can be linked by the Fc of the antibody to increase valency, or directly linked by a flexible short linker such as GSG.

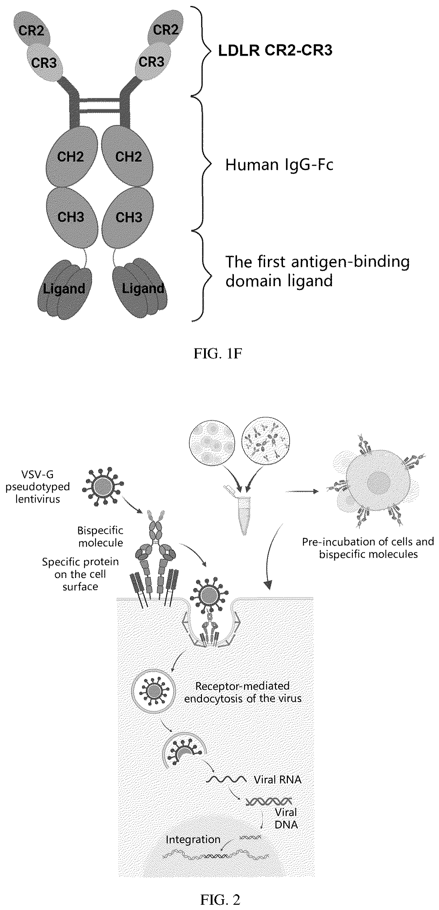

FIG. 2 shows a schematic diagram of cell infection using bispecific antigen-binding molecules. Firstly, the specific bispecific molecules at a certain concentration are incubated with cells at 4°C, so that the bispecific molecules bind to the specific proteins on the cell surface. The free bispecific molecules in the supernatant are discarded by centrifuging the cells; the cells are resuspended with VSV-G pseudotyped lentiviruses of a certain titer, and the VSV-G proteins on the viral surface bind to the CR2-CR3 domain of LDLRs; the bispecific molecules not only shorten the physical distance between the cells and the viruses, but also the proteins on the cell surface are endocytosed after being stimulated by the bispecific molecules, so that the viral particles are wrapped into the cells, and the low pH value in the endosomes promotes the conformational change of VSV-G proteins to fuse with the membrane, release genetic material into the cells, and achieve long-term gene transduction through reverse transcription and further integration into the cell genome; the process starting from the endocytosis is consistent with the traditional infection of VSV-G pseudotyped lentiviruses via the LDLR receptors.

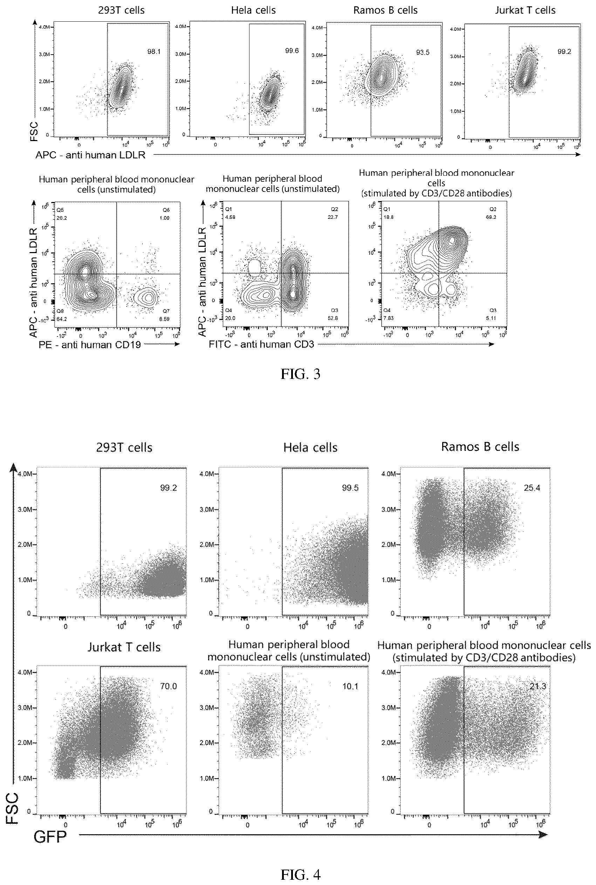

FIG. 3 shows the detection results of LDLR expression on the surfaces of different cell types by flow cytometry. By using flow cytometry antibodies against human low-density lipoprotein receptor LDLR, the expression was detected in different cell lines. Compared with common cell lines, the LDLR expression in human primary T cells not stimulated by CD3/CD28 antibodies was low, which was upregulated after CD3/CD28 antibody stimulation.
FIG. 4 shows the comparative analysis results of the infection efficiency of VSV-G pseudotyped GFP lentiviruses with equivalent titer on different cell types. The same titer of VSV-G pseudotyped GFP lentiviruses was used to infect different cell types, and the proportions of GFP-positive cells were detected by flow cytometry.

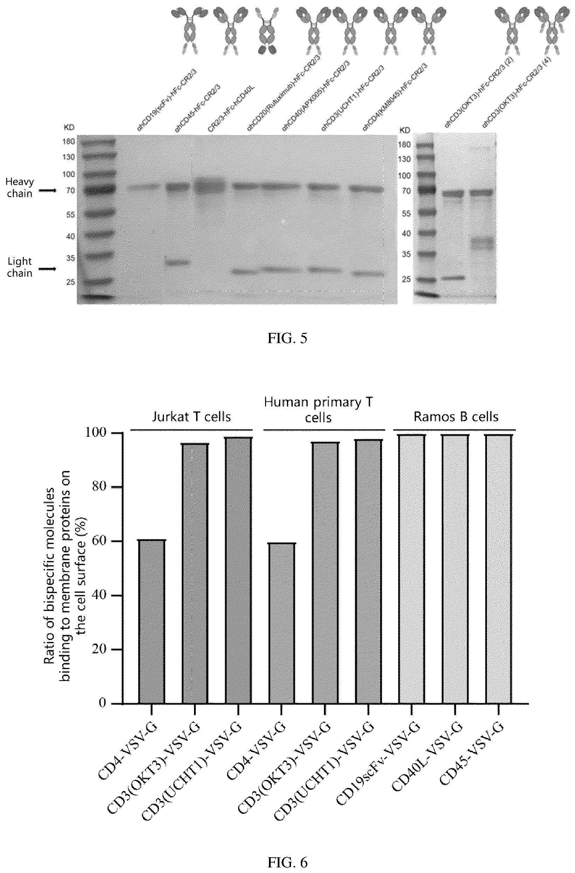

FIG. 5 shows the expression results of different bispecific molecules verified by SDS-PAGE. The expression of bispecific molecules was verified by SDS-PAGE, and the protein molecular weights of different bispecific molecules were consistent with the theoretical sizes.
FIG. 6 shows a bar chart of the binding ratio of bispecific molecules to membrane proteins on the surface of different cells. By incubating different bispecific molecules with different cell types respectively and using fluorescent secondary antibodies binding to the Fc fragment of the bispecific molecules to indicate the binding situation, it demonstrated that the expressed proteins could bind to specific cells via the first binding domain.
FIG. 7 shows a bar chart of the binding ratio of different bispecific molecules to 293T cells overexpressing VSV-G proteins. By incubating different bispecific molecules with 293T cells overexpressing VSV-G proteins respectively and using fluorescent secondary antibodies binding to the Fc fragment of the bispecific molecules to indicate the binding situation, it demonstrated that the expressed protein could bind to VSV-G proteins via the CR2-CR3 domain of human low-density lipoprotein receptor LDLR.
FIGS. 8A-C show the enhancing effect of bispecific molecules (targeting HLA) on the lentiviral infection efficiency in Ramos cells detected by flow cytometry.
FIGS. 9A-C show the enhancing effect of bispecific molecules (targeting HLA) on the lentiviral infection efficiency in Jurkat T cells detected by flow cytometry.
FIGS. 10A-C show the enhancing effect of bispecific molecules (targeting HLA) on the lentiviral infection efficiency in human peripheral blood mononuclear cells detected by flow cytometry.
FIGS. 11A-C show the enhancing effect of bispecific molecules (targeting CD19) on the lentiviral infection efficiency in Ramos cells detected by flow cytometry.
FIG. 12 shows the enhancing effect of bispecific molecules (targeting CD20) on the lentiviral infection efficiency in Ramos cells detected by flow cytometry.
FIG. 13 shows the enhancing effect of bispecific molecules (targeting CD45) on the lentiviral infection efficiency in Ramos cells detected by flow cytometry.
FIG. 14 shows the enhancing effect of bispecific molecules (targeting CD40) on the lentiviral infection efficiency in Ramos cells detected by flow cytometry.
FIG. 15 shows the enhancing effect of bispecific molecules (targeting CD3) on the lentiviral infection efficiency in Jurkat T cells detected by flow cytometry.
FIG. 16 shows the enhancing effect of bispecific molecules (targeting CD3) on the lentiviral infection efficiency in unstimulated human primary T cells detected by flow cytometry.
FIG. 17 shows the enhancing effect of bispecific molecules (targeting CD3) on the lentiviral infection efficiency in human primary T cells stimulated by CD3/CD28 antibodies detected by flow cytometry.
FIG. 18 shows the enhancing effect of bispecific molecules (targeting CD4) on the lentiviral infection efficiency in unstimulated human primary T cells detected by flow cytometry.

### SPECIFIC IMPLEMENTATIONS

To make the present disclosure easier to understand, certain terms are defined at first. Additional definitions are set forth throughout the detailed description.

### I. Definitions

Before describing the present disclosure in detail, it should be understood that the present disclosure is not limited to specific compositions or method steps, as these compositions or method steps can be altered. As used herein and in the appended claims, unless the context clearly indicates otherwise, the singular forms "a/an" and "the" include plural referents. The terms "a" (or "an"), and the terms "one or more" and "at least one" can be used interchangeably herein.

Furthermore, the use of "and/or" herein should be understood as the specific disclosure of each of the two designated features or components with or without the other. Thus, the term "and/or" used in phrases such as "A and/or B" is intended to include "A and B", "A or B", "A" (alone), and "B" (alone). Similarly, the term "and/or" used in phrases such as "A, B and/or C" is intended to include the following: A, B and C; A, B or C; A or C; A or B; B or C; A and C; A and B; B and C; A (alone); B (alone); and C (alone).

Unless otherwise defined, all technical and scientific terms used herein have the same meanings as commonly understood by those skilled in the art to which the present disclosure pertains. For example, the Concise Dictionary of Biomedicine and Molecular Biology, Juo, PeiShow, 2nd edition, 2002, CRC Press; the Dictionary of Cell and Molecular Biology, 3rd edition, 1999, Academic Press; and the Oxford Dictionary of Biochemistry and Molecular Biology, revised edition, 2000, Oxford University Press provide those skilled in the art general dictionaries for many terms used in the present disclosure.

Units, prefixes, and symbols are denoted in their accepted forms by International System of Units (SI). Numerical ranges include the numbers defining the range. Unless otherwise indicated, amino acid sequences are written from left to right in the direction from amino to carboxyl. The subheadings provided herein are not limitations of the various aspects, which can be obtained by referring to the specification as a whole. Therefore, the terms defined below are more fully defined by referring to the specification in its entirety.

It should be understood that when the term "comprising" is used to describe an aspect, other similar aspects described as "consisting of" and/or "primarily consisting of" are also provided.

Amino acids are referred to herein by their commonly known three-letter symbols or by the single-letter symbols recommended by the IUPAC-IUB Biochemical Nomenclature Commission. Similarly, nucleotides are referred to by their commonly accepted one-letter codes.

The term "antigen-binding molecule" as used herein refers to a molecule that specifically binds to an antigenic determinant in its broadest sense. Examples of antigen-binding molecules are immunoglobulins and derivatives thereof, such as fragments.

The term "antibody" refers to an immunoglobulin molecule that recognizes and specifically binds to a target such as proteins, polypeptides, peptides, carbohydrates, polynucleotides, lipids or their combinations by at least one antigen recognition site located within the variable region of the immunoglobulin molecule. As used herein, the term "antibody" encompasses complete polyclonal antibodies, complete monoclonal antibodies, antibody fragments (such as Fab, Fab', F(ab)2 and Fv fragment), single-chain variable fragments (scFv), disulfide-stabilized scFv, multispecific antibodies such as bispecific antibodies generated from at least two complete antibodies and/or antigen-binding fragments thereof, chimeric antibodies, humanized antibodies, human antibodies, fusion proteins comprising the antigenic determinant portion of an antibody, and immunoglobulin molecules comprising any other modifications of an antigen recognition site, provided that these antibodies exhibit the desired biological activity.

A conventional immunoglobulin is a tetramer composed of two heavy chains and two light chains, with a combined molecular weight of approximately 150 kDa. A typical antibody comprises at least two heavy (H) chains and two light (L) chains interconnected by disulfide bonds. Each heavy chain comprises a heavy chain variable region (abbreviated as VH herein) and a heavy chain constant region (abbreviated as CH herein). The heavy chain constant region comprises three domains (CH1, CH2 and CH3). Each light chain comprises a light chain variable region (abbreviated as VL herein) and a light chain constant region. The light chain constant region comprises one domain CL. These VH and VL regions can be further subdivided into hypervariable regions known as complementary-determining regions (CDRs), which are interspersed with more conserved regions called framework regions (FW). Each of VH and VL comprises three CDRs and four FWs arranged in the following order from the amino-terminus to the carboxyl-terminus: FW1, CDR1, FW2, CDR2, FW3, CDR3, FW4. The variable regions of the heavy and light chains comprise binding domains that interact with antigens. The constant regions of antibodies can mediate the binding of immunoglobulins to host cells or factors, including different cells of the immune system (e.g., effector cells) and the first component of the classical complement system (C1q).

The term "antigen-binding domain" refers to the part where antigens binding to molecules, i.e., one or more amino acid residues, which provides interaction with an antigen. For example, the antigen-binding domain of an antibody comprises amino acid residues from complementary-determining regions (CDRs). Examples of antigen-binding domains include, but are not limited to, Fab, Fab', F(ab')2 and single-chain Fv (scFv) fragments.

The term "scFv" refers to a fusion protein comprising at least one antibody fragment including the variable region of a light chain and at least one antibody fragment including the variable region of a heavy chain, wherein the light chain and heavy chain variable regions are adjoining (e.g., via a synthetic linker such as a short flexible polypeptide linker) and can be expressed as a single-chain polypeptide, and wherein the scFv retains the specificity of the complete antibody from which it is derived. Unless specified, the scFv can have the VL and VH variable regions in any order (e.g., relative to the N-terminus and C-terminus of the polypeptide), and the scFv can include VL-peptide linker-VH or VH-peptide linker-VL.

Five major classes of antibodies are known in the art: IgA, IgD, IgE, IgG and IgM, and their corresponding heavy chain constant domains are referred to as α, δ, ε, γ and µ, respectively. IgG and IgA can be further divided into different subclasses, e.g., IgG can be divided into IgG1, IgG2, IgG3, IgG4, and IgA can be divided into IgA1 and IgA2. The light chains of antibodies from any vertebrate species can be assigned to one of two distinct types, called kappa (κ) and lambda (λ), based on the amino acid sequence of their constant domains.

In the case of IgG, IgA and IgD antibodies, the constant region comprises three domains called CH1, CH2 and CH3 (IgM and IgE have a fourth domain, CH4). In the IgG, IgA and IgD classes, the CH1 and CH2 domains are separated by a flexible hinge region, which is a variable-length fragment rich in proline and cysteine. Each class of antibody further contains interchain and intrachain disulfide bonds formed by paired cysteine residues.

The term "Fc" is used herein to define the C-terminal region of the heavy chain of an immunoglobulin, i.e., the two dimer-forming polypeptide chains comprising the C-terminal constant region in the immunoglobulin heavy chain that are capable of stabilizing self-association. The term includes native sequence Fc regions and variant Fc regions. Although the boundaries of the Fc region of the IgG heavy chain may vary slightly, the Fc region of the human IgG heavy chain is generally defined as extending from Cys226 or Pro230 to the carboxyl-terminus of the heavy chain; for example, an IgG Fc domain comprises the IgG CH2 and IgG CH3 constant domains. Unless otherwise specified herein, the numbering of amino acid residues in the Fc regions or constant regions follow the EU numbering system, also known as the EU index, such as describing in Kabat et al., Sequences of Proteins of Immunological Interest, 5th edition, Public Health Service, National Institutes of Health, Bethesda, MD, 1991.

Among members of the Camelidae family, a significant proportion of serum antibodies are homodimeric IgG with a molecular weight of approximately 80 kDa (Hamers-Casterman et al., 1993, Nature, 363, 446-448). These heavy-chain immunoglobulins (Ig) comprise three domains. Their variable regions are referred to as VHHs (variable domains of heavy chain of heavy-chain antibody). Recombinant VHH (approximately 12 to 14 kDa) constitutes a complete antigen-binding domain and exhibits a broad antigen-binding spectrum. Their hypervariable regions are expanded and exhibit unique characteristics, such as the replacement of three to four hydrophobic framework residues (which interact with the VLs of conventional antibodies) with more hydrophilic amino acids. To stabilize the expanded CDRs, in addition to conventional disulfide bonds, VHH can have additional disulfide bonds between CDR1 and CDR3 of dromedaries, and between CDR2 and CDR3 of llamas (Harmsen and De Haard, 2007, Appl Microbiol Biotechnol., 77, 13-22; Muyldermans, 2001, J Biotechnol., 74, 277-302). The expanded CDR3 loop can adopt a convex conformation, while the conventional complementary site is restricted to concave or planar structures (Muyldermans, 2001, J Biotechnol., 74, 277-302). These features allow VHHs to recognize unique epitopes that are less immunogenic for conventional antibodies (Lafaye, 2009, Mol Immunol., 46, 695-704; Wernery, 2001, J Vet Med B Infect Dis Vet Public Health., 48, 561-568). Although VHH is defined as a monovalent antibody, which excludes any affinity effects by default, the biological activity measured as in vitro IC50 can be similar to that of conventional bivalent antibody molecules (Thys et al., 2010, Antiviral Res., 87, 257-264). In the present disclosure, "VHH antibody", "heavy chain variable region (VHH)", "VHH domain" and "nanobody VHH" can be used interchangeably.

A "monoclonal antibody" refers to a homogeneous population of antibodies that involves highly specific recognition and binding to a single antigenic determinant or epitope. This contrasts with polyclonal antibody, which typically includes different antibodies targeting different antigenic determinants.

The term "monoclonal antibody" encompasses both complete and full-length monoclonal antibodies, as well as antibody fragments (such as Fab, Fab', F(ab')2, Fv, single-chain variable fragments (scFv), fusion proteins comprising antibody portions and immunoglobulin molecules comprising any other modifications of the antigen recognition sites). Furthermore, "monoclonal antibody" refers to such antibodies prepared by any quantitative method, including but are not limited to via hybridomas, phage selection, recombinant expression and transgenic animals (e.g., expressing human antibodies in transgenic mice).

The term "humanized antibody" refers to an antibody derived from a non-human (e.g., murine) immunoglobulin, which has been engineered to contain minimal non-human (e.g., murine) sequences. Typically, a humanized antibody is a human immunoglobulin in which residues from the CDRs are replaced with residues from the CDRs of a non-human species (e.g., mouse, rat, rabbit or hamster) with promising specificity, affinity and capability (Jones et al., 1986, Nature, 321:522-525; Riechmann et al., 1988, Nature, 332:323-327; Verhoeyen et al., 1988, Science, 239:1534-1536). In some examples, FW residues of the human immunoglobulins are replaced with corresponding residues from antibodies of non-human species with promising specificity and/or affinity and/or capability.

The humanized antibody can be further modified by replacing additional residues in the FW regions and/or refining and optimizing specificity and/or affinity and/or capability of antibody within the replaced non-human residues. Generally, the humanized antibody will essentially comprise all of at least one and typically two or three variable domains, which comprise all or substantially all of the CDR regions corresponding to those of the non-human immunoglobulin, while all or substantially all of the FW regions are those of the common sequences of the human immunoglobulin. The humanized antibody can further comprise at least a portion of the immunoglobulin constant region or domain (Fc), typically that of the human immunoglobulin. Examples of methods for producing humanized antibodies are described in U.S. Patent NOs. 5,225,539 or 5,639,641.

The term "human antibody" refers to an antibody produced by a human or an antibody having an amino acid sequence corresponding to that of an antibody prepared by a human using any technique known in the art (e.g., recombinant expression in cultured cells or expression in transgenic animals). Thus, the term human antibody also encompasses an antibody having an amino acid sequence corresponding to that of an antibody originally produced by a human (or an engineered variant or derivative thereof) but expressed in a non-human system (e.g., production by chemical synthesis; recombinant expression in microbial, mammalian or insect cells; or expression in an animal subject). Therefore, an antibody obtained from a human subject or from a human cell (e.g., a hybridoma or cell line expressing a recombinant antibody or fragment thereof) and subsequently expressed in an animal such as a mouse is considered a human antibody. This definition of human antibody includes complete or full-length antibodies, fragments thereof, and/or antibodies comprising polynucleotides of at least one human heavy and/or light chain, such as, for example, an antibody comprising polynucleotides of a murine light chain and a human heavy chain.

The term "chimeric antibody" refers to an antibody in which the amino acid sequence of the immunoglobulin molecule is derived from two or more animal species. Typically, the variable regions of both the light and heavy chains correspond to the variable regions of antibodies derived from one species of mammals (e.g., mouse, rat, rabbit, etc.) with promising specificity and/or affinity and/or capability, while the constant regions are homologous to the sequences in the antibodies derived from another species (usually human) to avoid eliciting an immune response in these species.

The term "bispecific" refers to that an antigen-binding molecule is capable of specifically binding to two different antigenic determinants. Generally, a bispecific antigen-binding molecule comprises two antigen-binding sites, each of which is specific for a different antigenic determinant. In some embodiments, a bispecific antigen-binding molecule is capable of binding to two antigenic determinants simultaneously, particularly two antigenic determinants expressed on two different cells.

The term "antigen" refers to a substance recognized and specifically bound by an antibody or antibody-binding fragment. Broadly, an antigen can include any immunogenic fragment or determinant of a selected target, including single epitopes, multiple epitopes, single domains, multiple domains, or complete extracellular domains (ECD) or proteins. Peptides, proteins, glycoproteins, polysaccharides and lipids, as well as portions thereof and combinations thereof all can constitute antigens. Non-limiting exemplary antigens include tumor antigens or pathogen antigens, etc. "Antigen" can also refer to a molecule that elicits an immune response. Any form of antigens or cells or preparations containing the antigens can be used to generate the antibodies specific for the antigenic determinants. The antigens can be isolated full-length proteins, cell surface proteins (e.g., immunized by cells expressing at least a portion of the antigens on their surface), or soluble proteins (e.g., immunized by only the ECD portion of the proteins) or protein constructs (e.g., Fc antigens). The antigen can be produced in genetically modified cells. Any of the aforementioned antigens can be used alone or in combination with one or more immunogenicity-enhancing adjuvants known in the art. The DNA encoding the antigen can be genomic or non-genomic (e.g., cDNA) and can encode at least a portion of the ECD sufficient to elicit an immunogenic response. Any vector can be used to transform cells expressing the antigens, including but not are limited to adenoviral vectors, lentiviral vectors, plasmids and non-viral vectors such as cationic lipids.

The term "epitope" refers to a site on an antigen that specifically binds to an immunoglobulin or antibody. Epitopes can be formed by adjacent amino acids or non-adjacent amino acids juxtaposed by tertiary folding of the protein. Epitopes formed by adjacent amino acids usually remain after being exposed to denaturing solvents, while epitopes formed by tertiary folding are usually lost after treatment with denaturing solvents. Epitopes usually exist in a unique spatial conformation and include at least 3-15 amino acids. Methods for determining the epitope bound by a given antibody are well known in the art, including immunoblotting and immunoprecipitation assays, etc. Methods for determining the spatial conformation of an epitope include techniques in the art and those described herein, such as X-ray crystallography and two-dimensional nuclear magnetic resonance, etc.

The terms "polypeptide", "peptide" and "protein" are used interchangeably herein to refer to a polymer with amino acids of any length. The polymer can be linear, cyclic or branched. It can comprise modified amino acids, particularly conservatively modified amino acids, and it can be interrupted by non-amino acids. The term also includes modified amino acid polymers such as those that have been modified by sulfation, glycosylation, lipidation, acetylation, phosphorylation, iodination, methylation, oxidation, proteolytic processing, isoprenylation, racemization, selenylation, transfer-RNA-mediated amino addition such as arginylation, ubiquitination, or any other manipulation such as conjugation with a labeling component, etc. As used herein, the term "amino acid" refers to natural and/or unnatural or synthetic amino acids, including glycine as well as D or L optical isomers, and amino acid analogs and peptidomimetics. A polypeptide or amino acid sequence "derived from" a specified protein refers to the source of the polypeptide. The term also includes polypeptides expressed by the specified nucleic acid sequence.

The term "amino acid modification" (or "modified amino acid") includes amino acid replacements, insertions and/or deletions in a polypeptide sequence. "Amino acid replacement" or "replacement" or "substitution" herein refers to replacing an amino acid at a specific position in the parent polypeptide sequence with another amino acid, e.g., S32A replacement refers to the replacement of serine at position 32 with alanine.

"Specific binding" refers to that the binding is selective for the antigen and can be distinguished from unwanted or non-specific interactions. The capability of an antibody binding to a specific antigenic determinant can be determined by enzyme-linked immunosorbent assay (ELISA) or other techniques familiar to those skilled in the art, such as surface plasmon resonance (SPR) technology (analyzed on a BIAcore instrument) (Liljeblad et al., Glyco J, 17, 323-329 (2000)) and traditional binding assays (Heeley Endocr, Res, 28, 217-229 (2002)).

"Affinity" refers to the strength of the total non-covalent interactions between a single binding site of a molecule (e.g., a receptor) and its binding partner (e.g., a ligand). Unless otherwise indicated, "binding affinity" as used herein refers to the intrinsic binding affinity reflecting a 1:1 interaction between members of a binding pair (e.g., an antigen-binding portion and an antigen, or a receptor and its ligand). The affinity of molecule X for its partner Y is generally expressed as the dissociation constant (KD), which is the ratio of the dissociation and association rate constants (koff and kon, respectively). Thus, equivalent affinity can involve different rate constants as long as the ratio of the rate constants remains the same. Affinity can be measured by well-established methods known in the art, including those described herein. A specific method for measuring affinity is surface plasmon resonance (SPR).

The term "valency" as used herein refers to the presence of a specified number of antigen-binding regions in an antigen-binding molecule. Therefore, for example, the term "tetravalent bispecific antigen-binding molecule" refers to a bispecific antigen-binding molecule having four antigen-binding regions which can bind to the same or different antigens; in some examples, for example, "bivalent for a certain target" refers to that the bispecific antigen-binding molecule comprises two antigen-binding regions targeting a specific target. In some other examples, for example, a CD3 monovalent bispecific antigen-binding molecule refers to a bispecific antigen-binding molecule comprising one antigen-binding region targeting CD3.

In some examples of the present disclosure, the antibody is bivalent for both cell surface antigens and VSV-G. In other examples, the antibody may not be bivalent for cell surface antigens and VSV-G, such as monovalent, trivalent, tetravalent, etc.; the "valency" of the antibody for cell surface antigens and VSV-G can be the same or different.

It should be noted that the division of CDRs and FRs in the antibody variable regions of the present disclosure is determined according to the Kabat definition. Other naming and numbering systems, such as Chothia, IMGT or AHo, etc., are also known to those skilled in the art. Therefore, based on the antibody sequences of the present disclosure, humanized antibodies comprising one or more CDRs derived from any naming system are all explicitly included within the scope of the present disclosure.

The term "sequence identity" or "sequence similarity" or "sequence homology" refers to the percentage of amino acid residues in a candidate sequence that are identical to the amino acid residues in the reference polypeptide sequence after aligning the sequences (and introducing gaps if necessary) to achieve the maximum percentage of sequence identity, without considering any conservative substitutions as part of the sequence identity. Various methods in the art can be used for sequence alignment to determine the percentage of amino acid sequence identity, for example, using publicly available computer software such as BLAST, BLAST-2, ALIGN or MEGALIGN (DNASTAR) software. Those skilled in the art can determine appropriate parameters for the alignment, including any algorithms needed to obtain the maximum alignment for the full length of the sequences being compared.

"Antibody-dependent cell-mediated cytotoxicity" or "ADCC" refers to a form of cytotoxicity, wherein the secreted immunoglobulins that bind to Fc receptors (FcRs) presenting on certain cytotoxic cells (e.g., natural killer (NK) cells, neutrophils and macrophages) enable these cytotoxic effector cells to specifically bind to target cells having antigens and subsequently kill the target cells with cytotoxins. High-affinity IgG antibodies specific for the surface of target cells "arm" cytotoxic cells and it is required for such killing. The lysis of target cells is extracellular, requiring direct cell-to-cell contact and not involving complements. It should be considered that, in addition to antibodies, other proteins comprising an Fc region with the capability to specifically bind to antigen-bearing target cells (specifically Fc fusion proteins) will be capable of achieving cell-mediated cytotoxicity. For brevity, cell-mediated cytotoxicity resulting from the activity of Fc fusion proteins is referred to as ADCC activity herein.

The term "nucleic acid" or "polynucleotide" refers to deoxyribonucleic acids (DNA) or ribonucleic acids (RNA) and polymers thereof in either single-stranded or double-stranded form. Unless specifically limited, the term "nucleic acid" or "polynucleotide" also includes nucleic acids containing analogs of known natural nucleotides that have similar binding properties as the reference nucleic acids and are metabolized in a manner similar to naturally occurring nucleotides (see U.S. Patent No. 8,278,036 by Kariko et al., which discloses mRNA molecules in which uridine is replaced with pseudouridine, methods of synthesizing the mRNA molecules and methods for delivering therapeutic proteins *in vivo*). Unless otherwise indicated, specific nucleic acid sequences also implicitly include conservatively modified variants (e.g., degenerate codon replacements), alleles, orthologs, single nucleotide polymorphisms (SNPs) and complementary sequences, as well as the explicitly stated sequences.

The term "vector" refers to a construct capable of delivering, and in some aspects, expressing one or more genes or one or more sequences of interest in a host cell. Examples of vectors include, but are not limited to, viral vectors, naked DNA or RNA expression vectors, plasmids, cosmids or phage vectors, DNA or RNA expression vectors linked to cationic condensing agents, DNA or RNA expression vectors enveloped in liposomes and certain eukaryotic cells such as producer cells.

In the present disclosure, unless explicitly dictated otherwise, "activation", "stimulation", and "treatment" of a cell or receptor may have the same meaning, for example, a cell or receptor is activated, stimulated or treated with a ligand. "Ligands" include natural and synthetic ligands, such as cytokines, cytokine variants or analogs, muteins and binding compounds derived from antibodies (e.g., antibodies and binding fragments thereof). "Ligands" also include small molecules, such as peptidomimetics of cytokines and peptidomimetics of antibodies. "Activation" can refer to cell activation regulated by internal mechanisms as well as external or environmental factors. "Response" or "reaction", such as a response of cells, tissues, organs or organisms, includes a change in biochemical or physiological behavior, such as a change in the concentration, density, adhesion or migration of certain components within biological compartments (e.g., tissues, cells, organelles, etc.), in the rate of gene expression or the state of differentiation, which may be related to activation, stimulation or treatment.

The terms "host cell", "host cell line" and "host cell culture" can be used interchangeably, which refer to cells into which exogenous nucleic acids have been introduced, including the progeny of such cells. Host cells include "transformants" and "transformed cells", which include primary transformed cells and progeny derived therefrom, regardless of passage number. Progeny may not be entirely identical in nucleic acid content to the parent cells but may contain mutations. Mutant progeny that has the same functions or biological activities as those screened or selected in the originally transformed cells are included herein. Host cells are any type of cell systems that can be used to produce the bispecific antigen-binding molecules of the present disclosure. Host cells include cultured cells, such as mammalian cultured cells, including Jurkat cells, PBMC cells, A375 cells, U251 cells, U87 cells or hybridoma cells, yeast cells, insect cells and plant cells, etc., as well as cells comprised in transgenic animals, transgenic plants, or cultured plant or animal tissues.

The term "transfection" as used herein refers to the introduction of exogenous nucleic acids into eukaryotic cells. Transfection can be achieved by various means known in the art, including calcium phosphate-DNA co-precipitation, DEAE-dextran-mediated transfection, polybrene-mediated transfection, electroporation, microinjection, liposome fusion, lipofection, protoplast fusion, retroviral infection and biolistics.

The term "stable transfection" or "stably transfect" refers to the introduction and integration of exogenous nucleic acids, DNA or RNA into the genome of transfected cells. The term "stable transfectant" refers to cells in which foreign DNA is stably integrated into the genomic DNA.

Methods for producing and purifying antibodies and antigen-binding fragments are well known and can be found in the prior art, such as the Using Antibodies: A Laboratory Manual by the Cold Spring Harbor Laboratory, Chapters 5-8 and 15. The antibodies or the antigen-binding fragments thereof of the application are genetically engineered to add one or more human FR regions to non-human CDR regions. Human FR germline sequences can be obtained from the ImMunoGeneTics (IMGT) website (http://imgt.cines.fr) or from the Immunoglobulin FactsBook (2001) ISBN: 012441351.

The engineered antibodies or the antigen-binding fragments thereof of the present disclosure can be prepared and purified using conventional methods. For example, cDNA sequences encoding heavy and light chains can be cloned and recombined into expression vectors. Recombinant immunoglobulin expression vectors can be stably transfected into CHO cells. As a more recommended prior art, mammalian expression systems result in glycosylation of antibodies, particularly at the highly conserved N-terminus of the Fc region. Stable clones are obtained by expressing antibodies that specifically bind to human antigens. Positive clones are expanded in serum-free culture medium in bioreactors to produce antibodies. The culture medium with secreting antibodies can be purified and collected using conventional techniques. Antibodies can be filtered and concentrated using conventional methods. Soluble mixtures and polymers can also be removed using conventional methods such as molecular sieve and ion exchange.

### II. Bispecific antigen-binding molecules or fragments thereof

In one aspect of the present disclosure, a bispecific antigen-binding molecule (also referred to as a bispecific antibody in the present disclosure) or a fragment thereof is provided, comprising a first antigen-binding domain and a second antigen-binding domain, wherein the first antigen-binding domain is used for specifically binding to a cell surface antigen; the second antigen-binding domain is used for specifically binding to a vesicular stomatitis virus glycoprotein (VSV-G).

In the present disclosure, the first antigen-binding domain used for specifically binding to a cell surface antigen can be widely replaced to achieve specific targeting of different types of cells.

In some embodiments, the cell can be a cell with low expression of low-density lipoprotein receptors (LDL-Rs); as used herein, the terms "low expression" and "low expression level" can be interchangeable, and in application, they shall refers to a reduction of at least 2%, 3%, 4%, 5%, 6%, 7%, 8%, 9% or 10%, preferably at least 15% or 20%, more preferably 25%, 30%, 50%, 80%, 100% or more, compared with a "control" or "threshold". For example, Student's T-test can be repeatedly performed on genes with at least one expression intensity lower than the thresholds to determine significance.

In some embodiments, the cell can be a cell that do not express low-density lipoprotein receptor (LDL-R).

In some embodiments, the cell includes but are not limited to a T cell, a B cell, an NK cell or a hematopoietic stem cell (HSC).

In some embodiments, the T cell and B cell are primary cells.

In some embodiments, the T cell and B cell are resting cells. Resting cells have a lower LDL-R expression level compared to stimulated cells.

In some embodiments, the cell surface antigen is selected from a B cell surface antigen, an NK cell surface antigen, a hematopoietic stem cell surface antigen or a T cell surface antigen; exemplarily, the cell surface antigen is selected from any one of CD3, CD19, CD45, CD20, CD34, CD40, CD56, CD16, CD133, CD147, CD123, CD138, CD22, CD30, CD33, CD38, CD70, CD4, CD5, CD8A & CD8B and CD7.

In some embodiments, the second antigen-binding domain comprises CR2 and CR3 domains of a low-density lipoprotein receptor (LDL-R).

In some embodiments, the CR2 and CR3 domains are derived from human, horse or pig.

In some embodiments, the second antigen-binding domain comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.1.

In some embodiments, the amino acid sequence of the second antigen-binding domain is:

VTCKSGDFSCGGRVNRCIPQFWRCDGQVDCDNGSDEQGCPPKTCSQDEFRCHDGKCISR

QFVCDSDRDCLDGSDEASCPV (SEQ ID NO.1).

In some embodiments, the amino acid sequence of the second antigen-binding domain has one or several amino acid replacements, insertions and/or deletions compared with SEQ ID NO.1, and the modified second antigen-binding domain has a comparable or enhancing specific binding activity to vesicular stomatitis virus glycoproteins (VSV-G) over that of the wild type.

In some embodiments, "several" refers to 5 or fewer, more preferably 3 or fewer, and most preferably 2 or fewer. For example, the amino acid sequence of the second antigen-binding domain has 5, 4, 3, 2, or 1 amino acid residue replacements, insertions and/or deletions compared with SEQ ID NO.1.

In some embodiments, the CR2 and CR3 domains specifically bind to a vesicular stomatitis virus glycoprotein as a single-domain antibody.

In some embodiments, the bispecific antibody binds to a cell surface antigen, so that the cell surface is coated with the bispecific antibody molecules. During viral infection, the virus contacts the cell by recognizing the CR2 and CR3 domains on the bispecific antibody, and specific molecules on the cell surface such as CD19, CD3, CD45, etc., undergo clathrin-dependent endocytosis after being bound by the specific antibody. The endocytosis process mediates the entry of the virus into the target cell, ultimately achieving gene delivery.

In some embodiments, the first antigen-binding domain comprises a heavy chain (HC) variable region (VH) and/or a light chain (LC) variable region (VL).

As described herein, the first antigen-binding domain used for specifically binding to a cell surface antigen can comprise a heavy chain variable region and/or light chain variable region of any disclosed antibody targeting the cell surface antigen. Exemplarily, it can target any of the above cell surface antigens, such as but are not limited to the heavy chain variable region and/or light chain variable region of antibodies against CD3, CD19, CD45, CD20, CD34, CD40, CD56, CD16, CD133, CD147, CD123, CD138, CD22, CD30, CD33, CD38, CD70, CD4, CD5, CD8A & CD8B, CD7 or HLA.

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequences shown in SEQ ID NOs. 87-95;
and/or
a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequences shown in SEQ ID NOs. 96-104.

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.87; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.96. In some embodiments, the first antigen-binding domain can specifically bind to the cell surface antigen CD19.

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.88; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.97. In some embodiments, the first antigen-binding domain can specifically bind to the cell surface antigen CD3 (OKT3). In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.89; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.98. In some embodiments, the first antigen-binding domain can specifically bind to the cell surface antigen CD45.

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.90; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.99. In some embodiments, the first antigen-binding domain can specifically bind to the cell surface antigen CD34.

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.91; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.100. In some embodiments, the first antigen-binding domain can specifically bind to the cell surface antigen CD20.

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.92; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.101. In some embodiments, the first antigen-binding domain can specifically bind to the cell surface antigen CD40.

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.93; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.102. In some embodiments, the first antigen-binding domain can specifically bind to the cell surface antigen CD4.

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.94; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.103. In some embodiments, the first antigen-binding domain can specifically bind to the cell surface antigen CD3 (UCHT1).

In some embodiments, the first antigen-binding domain can comprise a heavy chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.95; and a light chain variable region with an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.104. In some embodiments, the first antigen-binding domain can specifically bind to the cell surface antigen HLA.

In some embodiments, the first antigen-binding domain can further comprise a heavy chain (HC) constant region (CH) or a fragment thereof and/or a light chain (LC) constant region (CL) or a fragment thereof.

In some embodiments, the heavy chain constant region or the fragment thereof is an IgG constant region; in some embodiments, the heavy chain constant region or the fragment thereof comprises at least the first constant region CH1 of IgG.

In some embodiments, the light chain constant region is a κ constant region or a λ constant region.

In some embodiments, the first antigen-binding domain includes at least one of Fab, Fab', F(ab')2, scFv or VHH.

In some embodiments, the first antigen-binding domain is a monoclonal antibody, a humanized antibody, a human antibody, a chimeric antibody, or an affinity-optimized antibody.

In some embodiments, the first antigen-binding domain further comprises an Fc domain.

In some embodiments, the Fc domain is derived from murine or human.

In some embodiments, the Fc domain further comprises at least one mutation capable of reducing or enhancing the ADCC activity of the bispecific antibody.

In some embodiments, the Fc domain comprises a modification that promotes the binding of the two chains of the Fc domain; in some embodiments, the Fc domain comprises a Knob chain and a Hole chain.

In some embodiments, the second antigen-binding domain is covalently linked to the carboxyl terminus of the heavy chain and/or light chain of the first antigen-binding domain;

In some embodiments, the second antigen-binding domain is covalently linked to the amino terminus of the heavy chain and/or light chain of the first antigen-binding domain;

In some embodiments, the second antigen-binding domain is covalently embedded in the polypeptide chain of the heavy chain and/or light chain of the first antigen-binding domain.

In some embodiments, when both the heavy chain and the light chain of the first antigen-binding domain are linked to the second antigen-binding domains, the linking sites of the second antigen-binding domain to the light chain and the heavy chain can be the same or different. For example, a second antigen-binding domain is linked to the carboxyl terminus of the heavy chain, and another second antigen-binding domain may be linked to the carboxyl terminus, amino terminus of the light chain, or embedded in the polypeptide chain of the light chain; or a second antigen-binding domain is linked to the amino terminus of the heavy chain, and another second antigen-binding domain may be linked to the carboxyl terminus, amino terminus of the light chain, or embedded in the polypeptide chain of the light chain, etc.

In some embodiments, the second antigen-binding domain is linked to the heavy chain and/or light chain of the first antigen-binding domain via a peptide linker; in some embodiments, the peptide linker is selected from L1, L2, L3 or L4, wherein the sequence of L1 is (G₄S)x, where x is any integer from 1 to 6 (SEQ ID NO.7), the sequence of L2 is (G₄S)xA, where x is any integer from 1 to 6 (SEQ ID NO.8), the sequence of L3 is EPKSSDKTHTCPPCP (SEQ ID NO.9), and the sequence of L4 is DKTHTCPPCP (SEQ ID NO.10); preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

In some embodiments, the bispecific antigen-binding molecule is tetravalent or hexavalent.

In some embodiments, the bispecific antigen-binding molecule is bivalent or tetravalent for two targets.

In some embodiments, the exemplary molecular structures of the bispecific antigen-binding molecules are shown in FIGS. 1A to 1F.

In one example, as shown in FIG. 1A, the first antigen-binding domain of the antibody has a Fab structure, and the second antigen-binding domain is linked to the C terminus of the heavy chain.

In one example, as shown in FIG. 1B, the first antigen-binding domain of the antibody has a Fab structure and an Fc domain, and the second antigen-binding domain is linked to the C terminus of the heavy chain (i.e., the C terminus of the heavy chain CH3).

The antibodies shown in FIGS. 1A and 1B are tetravalent, which are bivalent for both the cell surface antigens and VSV-G.

In some other examples, the second antigen-binding domain can also be linked to the C terminus of the light chain (e.g., light chain CL).

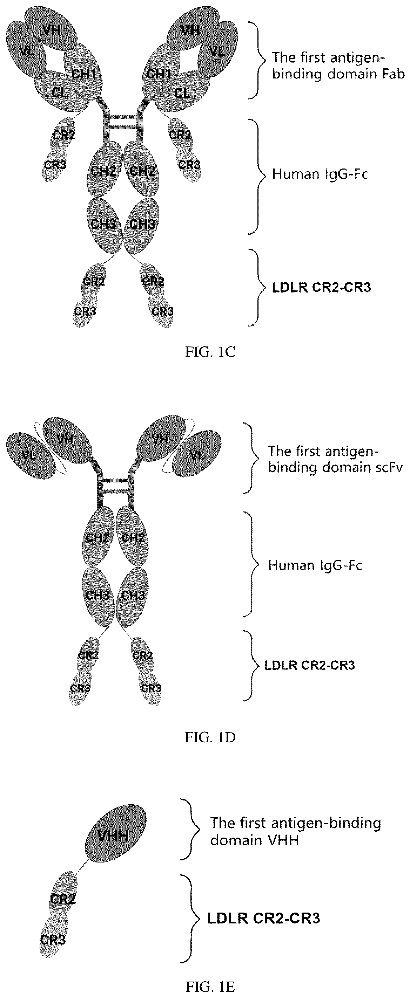

In another example, as shown in FIG. 1C, the first antigen-binding domain of the antibody has a Fab structure and an Fc domain, wherein the carboxyl termini of both the light chain and the heavy chain are linked to the second antigen-binding domains. In this case, the antibody is bivalent for the cell surface antigens and tetravalent for VSV-G. The inventors found that the binding ability of the bispecific antibody with such a structure to VSV-G was significantly improved.

The antibody shown in FIG. 1C is hexavalent, wherein being bivalent for the cell surface antigens and tetravalent for VSV-G.

In one example, as shown in FIG. 1D, the first antigen-binding domain of the antibody has an scFv structure and an Fc domain. The second antigen-binding domain is linked to the C-terminus of the Fc domain (i.e., heavy chain CH3).

In one example, as shown in FIG. 1E, the first antigen-binding domain of the antibody is a VHH antibody. The second antigen-binding domain is linked to the C-terminus of the first antigen-binding domain.

The antibody shown in FIG. 1D is bivalent, which is monovalent for both the cell surface antigens and VSV-G.

In one example, as shown in FIG. 1F, the first antigen-binding domain of the antibody has a ligand structure and an Fc domain. The second antigen-binding domain is linked to the N-terminus of the Fc domain.

The antibody shown in FIG. 1F is tetravalent, which is bivalent for both the cell surface antigens and VSV-G.

In the above examples, the second antigen-binding domain can be linked to the terminus of the first antigen-binding domain via a peptide linker.

In some embodiments, the bispecific antigen-binding molecule or the fragment thereof includes: a first polypeptide comprising a VH, a CH1, a CH2, a CH3, a CR2 and a CR3; and a second polypeptide comprising a VL and a CL;
wherein VH is the heavy chain variable region, VL is the light chain variable region; CH1, CH2 and CH3 are respectively the first, second and third constant regions of an IgG molecule, CL is the light chain constant region; CR2 and CR3 are the second and third CR domains of a low-density lipoprotein receptor (LDL-R).

In some embodiments, as shown in FIG. 1B, the first polypeptide comprises VH, CH1, CH2, CH3, CR2 and CR3 in the order from the N-terminus to the C-terminus; the second polypeptide comprises VL and CL in the order from the N-terminus to the C-terminus.

In some embodiments, the two polypeptides associate to form an antigen-binding site targeting the cell surface antigen; one end recognizes specific cell surface antigens (e.g., CD3 on the surface of T cells, CD19 on the surface of B cells, etc.) via the variable region of the antibody, and the other end of the Fc is coupled to the CR2 and CR3 domains of LDLR to bind to the VSV-G glycoproteins of the viruses.

In some embodiments, the bispecific antigen-binding molecule is tetravalent. For example, it comprises an antibody with four antigen-binding sites capable of binding to two different antigens (cell surface antigen and VSV-G).

In some embodiments, the bispecific antigen-binding molecule is bivalent for two targets (cell surface antigen and VSV-G).

In some embodiments, the first polypeptide comprises a peptide linker between CH3 and CR2; preferably, the peptide linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

In some embodiments, the first polypeptide comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.2; the second polypeptide comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.3.

In some embodiments, the first polypeptide comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.4; the second polypeptide comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.5.

In some embodiments, as shown in FIG. 1C, the second polypeptide further comprises CR2 and CR3 in the order from the N-terminus to the C-terminus. In some embodiments, the CR2 and CR3 are linked to the carboxyl terminus of CL.

In some embodiments, the second polypeptide further comprises a peptide linker between CL and CR2; preferably, the peptide linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

In some embodiments, the bispecific antigen-binding molecule or the fragment thereof comprises:
a first antigen-binding domain comprising an scFv, the scFv comprises a VH and a VL; and a second antigen-binding domain comprising a CR2 and a CR3; and optionally, CH2 and CH3 domains for linking the scFv and the second antigen-binding domain;
wherein VH is the heavy chain variable region, VL is the light chain variable region; CH2 and CH3 are respectively the second and third constant regions of an IgG molecule; CR2 and CR3 are the second and third CR domains of a low-density lipoprotein receptor (LDL-R).

In some embodiments, as shown in FIG. 1D, the bispecific antigen-binding molecule or the fragment thereof comprises scFv, CH2, CH3, CR2 and CR3 in the order from the N-terminus to the C-terminus; the scFv comprises VH and VL;

In some embodiments, the bispecific antigen-binding molecule is tetravalent.

In some embodiments, the bispecific antigen-binding molecule is bivalent for two targets.

In some embodiments, the bispecific antigen-binding molecule or the fragment thereof comprises a peptide linker between scFv and CH2; preferably, the linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

In some embodiments, the bispecific antigen-binding molecule or the fragment thereof comprises a peptide linker between CH3 and CR2; preferably, the peptide linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

In some embodiments, the bispecific antigen-binding molecule or the fragment thereof comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.6.

In some embodiments, the bispecific antigen-binding molecule or the fragment thereof comprises:
a first antigen-binding domain comprising a VHH antibody; and a second antigen-binding domain comprising a CR2 and a CR3; and optionally, CH2 and CH3 domains for linking the VHH and the second antigen-binding domain;
wherein CH2 and CH3 are respectively the second and third constant regions of an IgG molecule; CR2 and CR3 are the second and third CR domains of a low-density lipoprotein receptor (LDL-R).

In some embodiments, as shown in FIG. 1E, the bispecific antigen-binding molecule or the fragment thereof comprises VHH, CR2 and CR3 in the order from the N-terminus to the C-terminus.

In some embodiments, the bispecific antigen-binding molecule is bivalent.

In some embodiments, the bispecific antigen-binding molecule is monovalent for two targets.

In some embodiments, the bispecific antigen-binding molecule or the fragment thereof comprises a peptide linker between VHH and CR2; preferably, the linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

In some embodiments, the bispecific antigen-binding molecule or the fragment thereof comprises: a first antigen-binding domain comprising a natural ligand of a cell surface protein; a second antigen-binding domain comprising a CR2 and a CR3; and optionally, CH2 and CH3 domains for linking the natural ligand and the second antigen-binding domain;
wherein the first antigen-binding domain is used for specifically binding to a cell surface antigen; CH2 and CH3 are respectively the second and third constant regions of an IgG molecule; CR2 and CR3 are the second and third CR domains of a low-density lipoprotein receptor (LDL-R).

In some embodiments, as shown in FIG. 1F, the bispecific antigen-binding molecule or the fragment thereof comprises CR2, CR3, CH2, CH3 and a natural ligand in the order from the N-terminus to the C-terminus.

In some embodiments, the natural ligand is CD40 ligand. In some embodiments, the amino acid sequence of the CD40 ligand is shown in SEQ ID NO.106.

In some embodiments, the bispecific antigen-binding molecule is tetravalent.

In some embodiments, the bispecific antigen-binding molecule is bivalent for two targets.

In some embodiments, the bispecific antigen-binding molecule or the fragment thereof comprises a peptide linker between CR3 and CH2; preferably, the linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

In some embodiments, given the sequence information of the heavy chain variable region (VH) and/or light chain variable region (VL) of the first antigen-binding domain, the sequence information of the second antigen-binding domain (CR2 and CR3), and the connection relationship between the first antigen-binding domain and the second antigen-binding domain, those skilled in the art can obtain the full-length sequence of the antibody based on the conventional sequences of CH1, CH2, CH3, CL, etc. in the prior art (for example, by directly replacing VH and VL in the full-length antibody sequence provided in the present disclosure with the heavy chain and/or light chain variable regions (VH and/or VL) of the antigen-binding domains that bind to other target cell surface antigens to obtain bispecific antibodies that bind to other target cell surface antigens), as well as the nucleic acid sequences encoding the antibody.

In another aspect, the present disclosure provides the use of the bispecific antigen-binding molecule or the fragment thereof of the present disclosure for mediating a gene transduction of a receptor-independent lentiviral vector; preferably, the subject of the gene transduction is a cell with low or no expression of low-density lipoprotein receptors; preferably, the subject of the gene transduction is selected from a T cell, a B cell, an NK cell or a hematopoietic stem cell; preferably, the T cell and B cell are primary cells; preferably, the T cell and B cell are resting cells.

### III. Preparation of bispecific antigen-binding molecules or fragments thereof

The bispecific antibodies of the present disclosure can be prepared according to methods known in the art. For example, the bispecific antibodies of the present disclosure can be produced such as those hybridoma methods described in Kohler and Milstein (1975) Nature 256: 495.

Using this hybridoma method, mice, hamsters or other appropriate host animals are immunized as described above to induce production by lymphocytes of the antibodies that will specifically bind to the immunizing antigens. Lymphocytes can also be immunized *in vitro.* After immunization, these lymphocytes are isolated and fused with suitable myeloma cell lines using, for example, polyethylene glycol to form hybridoma cells, which can then be selected from unfused lymphocytes and myeloma cells. Hybridomas producing monoclonal antibodies specifically for the selected antigens, as determined by immunoprecipitation, immunoblotting or by *in vitro* binding assays (e.g., radioimmunoassay (RIA); enzyme-linked immunosorbent assay (ELISA)), can then be propagated using standard methods (Goding, Monoclonal Antibodies: Principles and Practice, Academic Press, 1986) in *in vitro* culture or *in vivo* as ascites tumors in animals. These monoclonal antibodies can then be purified from the culture medium or ascites fluid relative to the polyclonal antibodies as described above.

Recombinant DNA methods such as those described in U.S. Patent No. 4,816,567 can also be used to prepare the bispecific antibodies of the present disclosure. Polynucleotides encoding monoclonal antibodies are isolated from mature B cells or hybridoma cells, for example, by RT-PCR using oligonucleotide primers that specifically amplify the genes encoding antibody heavy and light chains, and their sequences are determined using conventional procedures. The isolated polynucleotides encoding the heavy and light chains are then cloned into suitable expression vectors. When transfected, these expression vectors enter host cells that do not otherwise produce immunoglobulin proteins, such as *E. coli* cells, simian COS cells, Chinese hamster ovary (CHO) cells or myeloma cells, and monoclonal antibodies are produced by these host cells. Similarly, as described, recombinant antibodies of the desired species or molecules thereof comprising antigen-binding fragments can be isolated from phage display libraries expressing the CDRs of the desired species (McCafferty et al., Nature 348: 552-554 (1990); Clarkson et al., Nature 352: 624-628 (1991); and Marks et al., J. Mol. Biol. 222: 581-597 (1991)).

One or more polynucleotides encoding the bispecific antibodies of the present disclosure can be further modified using recombinant DNA technology in various ways to produce alternative bispecific antibodies of the present disclosure. In some aspects, for example, the constant domains of the light and heavy chains of a mouse monoclonal antibody can be replaced with (1) those of, for example, a human antibody to produce a chimeric antibody or (2) a non-immunoglobulin polypeptide to produce a fusion antibody. In some aspects, the constant regions are truncated or removed to produce desired antibody fragments of the monoclonal antibody. Site-directed or high-density mutagenesis of the variable regions can be used to optimize the specificity, affinity, etc. of the monoclonal antibodies.

In certain aspects, the bispecific antibodies of the present disclosure are human antibodies or antigen-binding fragments thereof. Human antibodies can be prepared directly using different techniques known in the art. Immortalized human B lymphocytes capable of generating *in vitro* immunization or isolated from immunized individuals producing antibodies against the target antigens can be generated (see, e.g., Cole et al., Monoclonal Antibodies and Cancer Therapy, Alan R. Liss, p. 77 (1985); Boemer et al., J. Immunol. 47: 86-95 (1991); and U.S. Patent No. 5,750,373). One or more cDNAs encoding antibodies in immortalized human B lymphocytes can then be prepared and inserted into expression vectors and/or heterologous host cells for use in expressing non-naturally occurring recombinant forms of the antibodies.

Similarly, the bispecific antibodies or the antigen-binding fragments thereof of the present disclosure can be selected from phage libraries, wherein the phage library expresses human antibodies or fragments thereof as fusion proteins with heterologous phage proteins, as described, for example, in Vaughan et al., Nat. Biotech. 14: 309-314 (1996); Sheets et al., Proc. Natl. Acad. Sci. USA95: 6157-6162 (1998); Hoogenboom and Winter, J. Mol. Biol. 227: 381 (1991) and Marks et al., J. Mol. Biol. 222: 581 (1991)). Techniques for generating and using antibody phage libraries are also described in U.S. Patent Nos. 5,969,108; 6,172,197; 5,885,793; 6,521,404; 6,544,731; 6,555,313; 6,582,915; 6,593,081; 6,300,064; 6,653,068; 6,706,484; and 7,264,963, each of which is incorporated by reference in its entirety.

Affinity maturation strategy and chain shuffling strategy (Marx et al., BioTechnology 10: 779-783 (1992), which is incorporated by reference in its entirety) are known in the art and can be used to generate high-affinity human antibodies or antigen-binding fragments thereof.

In some aspects, the bispecific antibodies of the present disclosure can be humanized antibodies. Methods for engineering, humanizing, or resurfacing non-human or human antibodies can also be used, which are also known in the art. Humanized, resurfaced or similarly engineered antibodies can have one or more amino acid residues from non-human sources such as, but are not limited to, mice, rats, non-human primates or other mammals. These non-human amino acid residues are replaced with residues often referred to as "import" residues, which are typically taken from "import" variable, constant or other domains of known human sequences. Such imported sequences can be used to reduce immunogenicity or reduce, enhance or improve binding, affinity, on-rate, off-rate, avidity, specificity, half-life or any other suitable characteristic known in the art. Generally, CDR residues are directly and primarily involved in influencing antigen (such as cell surface antigen) binding. Thus, part or all of the non-human or human CDR sequences are retained while non-human sequences of variable and constant regions can be replaced with human or other amino acids. In certain aspects, human CDRs are inserted into non-human antibody scaffolds to prepare antibodies with reduced immunogenicity in animal model systems, such as "murinized" antibodies.

The bispecific antibodies of the present disclosure can optionally be humanized, resurfaced, or engineered, while retaining high affinity for cell surface antigens and other favorable biological properties. To achieve this goal, humanized (or human) or engineered antibodies and resurfaced antibodies can optionally be prepared by using a method that analyzes the parental sequences and different conceptual humanized and engineered products by employing three-dimensional models of the parental, engineered and humanized sequences. Three-dimensional immunoglobulin models are commonly available and well-known to those skilled in the art.

Computer programs that illustrate and display probable three-dimensional conformational structures of selected candidate immunoglobulin sequences are available. Inspection of these displays allows analysis of the likely role of residues in the function of the candidate immunoglobulin sequences, i.e., analysis of residues that influence the ability of the candidate immunoglobulins to bind to the first target (such as CD3, CD19, CD45, etc.) and/or the second target (VSV-G). In this way, framework residues can be selected from the consensus and import sequences and combined to achieve desired antibody characteristics, such as increased affinity for one or more target antigens.

Humanization, resurfacing or engineering of the bispecific antibodies of the present disclosure can be performed using any known method, such as but are not limited to those described in the following references: Jones et al., Nature 321: 522 (1986); Riechmann et al., Nature 332: 323 (1988); Verhoeyen et al., Science 239: 1534 (1988)); Sims et al., J. Immunol. 151: 2296 (1993); Chothia and Lesk, J. Mol. Biol. 196: 901 (1987), Carter et al., Proc. Natl. Acad. Sci. USA 89: 4285 (1992); Presta et al., J. Immunol. 151: 2623 (1993), U.S. Patent NOs. 5,639,641; 5,723,323; 5,976,862; 5,824,514; 5,817,483; 5,814,476; 5,763,192; 5,723,323; 5,766,886; 5,714,352; 6,204,023; 6,180,370; 5,693,762; 5,530,101; 5,585,089; 5,225,539; 4,816,567; 7,557,189; 7,538,195; and 7,342,110; WO90/14443; WO90/14424; WO90/14430; and EP229246, each of which is incorporated herein by reference in its entirety, including references cited therein.

In certain aspects, fragments of the bispecific antibodies of the present disclosure are provided.

Various techniques are known for producing antibody fragments. Traditionally, these fragments are derived by proteolytic digestion of complete antibodies (e.g., Morimoto et al., J. Biochem. Biophy. Methods 24: 107-117 (1993); Brennan et al., Science, 229: 81 (1985)). In certain aspects, the bispecific antibody fragments of the present disclosure are recombinantly produced. All Fab, Fv, and scFv antibody fragments can be expressed in and secreted from *E. coli* or other host cells, thereby allowing the production of large amounts of these fragments. Such antibody fragments can also be isolated from the antibody phage libraries discussed above. These antibody fragments can also be linear antibodies as described in U.S. Patent NO. 5,641,870. Other techniques for producing antibody fragments will be apparent to those skilled in the art.

Techniques suitable for producing single-chain antibodies (scFv) can be found, for example, in U.S. Patent NO. 4,946,778. Additionally, methods suitable for constructing Fab expression libraries can be found, for example, in Huse et al., Science 246: 1275-1281 (1989), to allow rapid and efficient identification of monoclonal Fab fragments with promising specificity for cell surface antigens (such as CD3, CD19, CD45, etc.), or derivatives, fragments, analogs or homologs thereof. Antibody fragments can be produced by techniques in the art. These antibody fragments include but not are limited to: (a) F(ab')2 fragments produced by pepsin digestion of antibody molecules; (b) Fab fragments produced by reducing the disulfide bridge bonds of F(ab')2 fragments; (c) Fab fragments produced by treating antibody molecules with papain and reducing agents; and (d) Fv fragments.

In some aspects, particularly in the case of antibody fragments, the antibody or the antigen-binding fragment thereof can be modified to increase its serum half-life. This can be achieved, for example, by following ways: incorporating salvage receptor binding epitopes into the antibody or antibody fragment by mutating appropriate regions in the antibody or antibody fragment or by incorporating the epitope and then fusing to the peptide tags at either end or in the middle of the antibody or antibody fragment (e.g., by DNA or peptide synthesis), or by YTE mutations. Other methods for increasing the serum half-life of the antibody or the antigen-binding fragment thereof (e.g., conjugation to a heterologous molecule such as PEG) are well-known in the art.

It should be noted that in certain aspects, bispecific antibody molecules can be engineered to directly fuse the CH3 domain to the hinge region of the correspondingly modified antibody or fragment thereof. In other constructs, peptide spacers can be inserted between the hinge region and the modified CH2 and/or CH3 domains. For example, compatible constructs can be expressed, wherein the CH2 domain has been deleted and the remaining CH3 domain (modified or unmodified) is linked to the hinge region using a spacer of 5-20 amino acids. Such a spacer can be added, for example, to ensure that regulatory elements of the constant domain remain free and accessible or to ensure that the hinge region remains flexible. However, it should be noted that amino acid spacers can be proved immunogenic and elicit an undesirable immune response against the constructs in some cases. Therefore, in certain aspects, any spacer added to the construct will be relatively non-immunogenic, or even omitted altogether, to maintain the desired biochemical qualities of the modified antibody.

The bispecific antibodies of the present disclosure can be expressed from one or more vectors. For example, in some embodiments, the first polypeptide is expressed by one vector and the second polypeptide is expressed by a second vector. In some embodiments, the first polypeptide and the second polypeptide are expressed by one vector. In some embodiments, the expression efficiency of the polypeptide is enhanced and/or increased by using only one vector. In some embodiments, the production efficiency of the bispecific antibody is enhanced and/or increased by expressing only two polypeptides. In some embodiments, the production efficiency of the bispecific antibody is enhanced and/or increased by expressing only two polypeptides compared to expressing three or more polypeptides. In some embodiments, the formation of active antigen-binding sites is enhanced and/or increased by expressing only two polypeptides. In some embodiments, the formation of active antigen-binding sites is enhanced and/or increased by expressing two polypeptides compared to expressing three or more polypeptides. In some embodiments, the production efficiency of the bispecific antibody is enhanced and/or increased by forming homodimeric molecules compared to forming heterodimeric molecules. In some embodiments, the stability of the bispecific antibody is enhanced and/or increased by forming homodimeric molecules compared to forming heterodimeric molecules.

In another aspect, the present disclosure provides a polynucleotide encoding the bispecific antigen-binding molecule or the fragment thereof of the present disclosure.

The polynucleotides of the present disclosure can be in the form of RNA or DNA. DNA includes cDNA, genomic DNA, and synthetic DNA; and can be double-stranded or single-stranded, and if single-stranded, can be the coding strand or the non-coding (antisense) strand. In certain aspects, the DNA is cDNA used to produce non-naturally occurring recombinant antibodies.

In certain aspects, these polynucleotides are isolated. In certain aspects, these polynucleotides are substantially pure. In certain aspects, these polynucleotides comprise coding sequences of mature polypeptides fused in the same reading frame as the polynucleotides (natural or heterologous) that, for example, assist in expressing and secreting the peptides from host cells (e.g., as a leader sequence of the secretory sequence that controls peptide transport from the cell). Polypeptide with leader sequences is preproteins and may have leader sequences cleaved by the host cells to form the mature form of the peptides. These polynucleotides can also encode a preprotein that is a mature protein plus additional 5' amino acid residues. In certain aspects, these polynucleotides are altered to optimize codon usage for a particular host cell.

In certain aspects, these polynucleotides comprise coding sequences of mature antibody molecules fused in the same reading frame as heterologous tag sequences that allow, for example, the purification of the encoded polypeptides. For example, the tag sequences can be, for instance, His6 tags supplied by pQE-9 vectors to provide the purification of the mature polypeptides fused to the tags in the case of bacterial hosts. In other aspects, the tag sequences can be, for example, hemagglutinin (HA) tags derived from influenza hemagglutinin proteins when using a mammalian host (e.g., COS-7 cells).

The present disclosure further relates to variants of the polynucleotides encoding, for example, the bispecific antibody fragments, analogs and derivatives of the present disclosure.

These polynucleotide variants can contain alterations in the coding region, non-coding region or both. In some aspects, these polynucleotide variants contain alterations that result in silent replacements, additions or deletions but do not alter the characteristics or activities of the encoded polypeptides. In some aspects, nucleotide variants are produced by silent replacements due to the degeneracy of the genetic code. Polynucleotide variants can be produced for various reasons, such as to optimize codon expression for a specific host (changing codons in human mRNA to those preferred by a bacterial host such as *E. coli*).

In some aspects, DNA sequences encoding the bispecific antibodies of the present disclosure can be constructed by chemical synthesis, for example, using an oligonucleotide synthesizer. Such oligonucleotides can be designed based on the amino acid sequences of the desired polypeptides and selecting those codons of the host cells in which the favorable recombinant polypeptides of interest will be produced. Standard methods can be applied to synthesize isolated polynucleotide sequences encoding the isolated polypeptides of interest. For example, a reverse-translated gene can be constructed using a complete amino acid sequence. Additionally, DNA oligomers comprising nucleotide sequences encoding the specific isolated polypeptides can be synthesized. For example, several small oligonucleotides encoding portions of the desired polypeptides can be synthesized and then ligated. Each oligonucleotide typically comprises 5' or 3' overhangs for complementary assembly.

Once assembled (by synthesis, site-directed mutagenesis or another method), the polynucleotide sequences encoding the specific isolated polypeptides of interest are inserted into the expression vector and operably linked to expression control sequences suitable for expressing the protein in the promising host. Appropriate assembly can be confirmed, for example, by nucleotide sequencing, restriction mapping and expression of the biologically active polypeptides in a suitable host. As is well known in the art, in order to achieve high-level expression level of the transfected genes in the host, the genes must be operably linked to transcriptional and translational expression control sequences that are functional in the selected expression host.

In another aspect, the present disclosure provides a recombinant vector comprising the polynucleotide of the present disclosure.

In certain aspects, the recombinant vectors are replicable DNA constructs comprising polypeptide chains encoding the antibodies and/or the antigen-binding fragments thereof of the present disclosure, synthetic or cDNA-derived DNA fragments operably linked to suitable transcriptional or translational regulatory elements derived from mammalian, microbial, viral or insect genes. A transcriptional unit typically includes an assembly of: (1) genetic elements or multiple elements that regulate gene expression, such as transcription promoters or enhancers, (2) structural or encoding sequence that are transcribed into mRNA and translated into proteins, and (3) appropriate transcription and translation initiation and termination sequences. Such regulatory elements can include operator sequences that control the transcription.

The ability to replicate in a host, typically conferred by the origin of replication, and selection genes to facilitate recognition of transformants can additionally be incorporated. DNA regions are operably linked when they are functionally related to each other. For example, the DNA of the signal peptide (secretory leader) is operably linked to the DNA of the polypeptide if it is expressed as a precursor involved in the secretion of the polypeptide; the promoter is operably linked to the encoding sequence if it controls the transcription of the sequence; or the ribosome binding site is operably linked to the encoding sequence if it is positioned to permit translation. Structural elements intended for use in yeast expression systems include leader sequences that enable extracellular secretion of translated proteins by the host cells. Alternatively, in the case of expressing recombinant proteins without leader or transport sequences, it may include a N-terminal methionine residue. This residue can optionally be subsequently cleaved from the expressed recombinant protein to provide the final product.

The selection of expression control sequences and expression vectors depends on the selection of hosts. A wide variety of expression host/vector combinations can be employed. Useful expression vectors for eukaryotic hosts include, for example, vectors comprising expression control sequences from SV40, bovine papillomavirus, adenovirus and cytomegalovirus. Useful expression vectors for bacterial hosts include known bacterial plasmids, such as plasmids from *E.coli* including pCR1, pBR322, pMB9 and derivatives thereof, plasmids within a wider host range such as M13 and filamentous single-stranded DNA phage.

**In** another aspect, the present disclosure provides a host cell comprising the polynucleotide or the recombinant vector of the present disclosure, including prokaryotic cells, yeast, insect or higher eukaryotic cells under the control of appropriate promoters. Prokaryotic cells include gram-negative or gram-positive organisms, such as *E.coli* or bacteria. Higher eukaryotic cells include established cell lines originated form mammalians as described below. Cell-free translation systems can also be employed. Suitable cloning and expression vectors for use with bacterial, fungal, yeast and mammalian cell hosts are described by Pouwels et al. (Cloning Vectors: A Laboratory Manual, Elsevier, N.Y., 1985), the relevant disclosure of which is hereby incorporated by reference.

Additional information of methods for protein production (including antibody production) can be found in, for example, U.S. Publication NO. 2008/0187954, U.S. Patent NOs. 6,413,746 and 6,660,501, and International Patent Publication NO. WO 04009823, each of which is hereby incorporated by reference in its entirety.

Different mammalian or insect cell culture systems can also be advantageously employed to express the bispecific antibody molecules or the antigen-binding fragments thereof of the present disclosure. Expression of recombinant proteins in mammalian cells can be performed because such proteins are usually correctly folded, appropriately modified and fully functional. Examples of suitable mammalian host cell lines include HEK-293 and HEK-293T, the COS-7 monkey kidney cell line described by Gluzman (Cell 23: 175, 1981); and other cell lines including, for example, L cells, C127, 3T3, Chinese Hamster Ovary (CHO), NSO, HeLa and BHK cell lines. Mammalian expression vectors can comprise non-transcribed elements such as an origin of replication, suitable promoters and enhancers linked to the genes to be expressed, and other 5' or 3' flanking non-transcribed sequences, as well as 5' or 3' non-translated sequences such as necessary ribosome binding sites, polyadenylation sites, splice donor and acceptor sites, and transcribed termination sequences. The baculovirus system for producing heterologous proteins in insect cells is reviewed by LuckoW and Summers, Biotechnology 6: 47 (1988).

Antibodies or antigen-binding fragments thereof produced by transformed hosts can be purified according to any suitable method. Such standard methods include, for example, chromatography (e.g., ion exchange, affinity and size exclusion chromatography), centrifugation, differential solubility or any other standard technique by protein purification. Affinity tags such as hexahistidine, maltose-binding domain, influenza shell sequence, glutathione S-transferase, etc., can be attached to proteins to allow simple purification by passage over an appropriate affinity column. Isolated proteins can also be physically characterized using, for example, proteolysis, nuclear magnetic resonance or X-ray crystallography.

For example, supernatants from systems that secrete recombinant proteins into the culture medium can first be concentrated using commercially available protein concentration filters, such as Millipore ultrafiltration units. After the concentration step, the concentrate can be applied to the suitable purification matrix. Alternatively, an anion exchange resin can be employed, such as matrices or substrates with diethylaminoethyl (DEAE) groups as side chains. These matrices can be acrylamide, agarose, dextran, cellulose or other types commonly used for protein purification. Alternatively, a cation exchange step can be employed.

Suitable cation exchangers include various insoluble matrices containing sulfopropyl or carboxymethyl groups. Finally, one or more reverse-phase high-performance liquid chromatography (RP-HPLC) steps using hydrophobic RP-HPLC media (e.g., silica gel with methyl or other aliphatic groups as side chains) can be employed to further purify the antibody molecules. Some or all of the aforementioned purification steps can also be employed in different combinations to provide homogeneous recombinant proteins.

Bispecific antibodies or antigen-binding fragments thereof of the present disclosure produced in bacterial cultures can be isolated, for example, by initial extraction from cell precipitates, then by one or more concentration, salting-out, aqueous ion exchange or size exclusion chromatography steps. High-performance liquid chromatography (HPLC) can be employed for the final purification step. Microbial cells used in expressing the recombinant proteins can be disrupted by any convenient method, including freeze-thaw cycles, ultrasound, mechanical disruption or the use of cell lysis agents.

Methods known in the art for purifying antibodies and other proteins also include, for example, those described in U.S. Patent Publication NOs. US20080312425, US20080177048, and US20090187005, each of which is hereby incorporated by reference in its entirety.

In another aspect, the present disclosure provides a method for transducing a target gene into a subject using a lentiviral vector, comprising administering the bispecific antigen-binding molecule or the fragment thereof of the present disclosure to the subject.

The lentiviral vectors in the present disclosure can encode exogenous genes in their genomic RNA. Recombinant viral vectors comprising exogenous genes can be obtained by inserting exogenous genes into the lentiviral vector genome. The "exogenous gene" referred to in the present disclosure can be any target gene that needs to be expressed in cells, and can be a gene encoding a natural protein or a gene of a protein modified by deleting, replacing or inserting amino acid residues in the natural protein. Exogenous genes can be inserted into any target positions in the non-coding regions of proteins in the viral genome. The target gene is transferred into the subject's cells via the lentiviral vectors and expressed in the subject's cells; furthermore, the expression of the target gene in the subject's cells can play a role in preventing and/or treating diseases. Those skilled in the art can select the target gene carried by the lentiviral vectors as needed, which is not limited herein.

### Examples

Plasmid Vector Construction
(1) For CD19scfv-VSV-G bispecific antibodies, the vector construction involved inserting the DNA encoding the scfv fragment of the anti-human CD19 before the DNA encoding the Fc fragment of the human antibody, inserting the DNA fragment encoding CR2-CR3 of human LDL-R after the Fc fragment of the human antibody, and then inserting the fully linked sequence of the three fragments into an expression vector that could be expressed in mammalian cells, wherein the expression vector MG-HSP-v1 in mammalian cells contains a CMV promoter, which can initiate the expression of downstream genes in mammalian cells.

The scfv fragment of anti-human CD19 was obtained by optimizing the DNA sequences according to the article (DOI:10.1182/blood-2010-04-281931) using the codon optimization tool of GenScript (https://www.genscript.com/gensmart-free-gene-codon-optimization.html) and then synthesizing them. Its nucleotide sequence is shown as SEQ ID NO.56. The Fc fragment of anti-human CD19 was obtained by optimizing the DNA sequences according to the protein database UniProt (P01857·IGHG1_HUMAN) using the codon optimization tool of GenScript (https://www.genscript.com/gensmart-free-gene-codon-optimization.html) and then synthesizing them. Its nucleotide sequence is shown as SEQ ID NO.57. The DNA encoding human LDL-R CR2-CR3 was obtained by optimizing the DNA sequences according to the protein database UniProt (P01130·LDLR_HUMAN) using the codon optimization tool of GenScript (https://www.genscript.com/gensmart-free-gene-codon-optimization.html) and then synthesizing them. Its nucleotide sequence is shown as SEQ ID NO.58.

The three fragments were ligated together using overlap extension polymerase chain reaction. The primers used are shown in Table 1 below. The expression vector MG-HSP-v1 (whose nucleotide sequence is shown as SEQ ID NO.55) was treated with the BsaI restriction endonuclease (NEB, Cat# R3733L) to linearize it.

**Table 1**

| Primer name | Sequence (5' to 3') |
|---|---|
| Upstream primer for the DNA sequence of anti-human CD19SCFV: | gcaaccggtgtacattctgatatacagatgacgcagac (SEQ ID NO.15) |
| Downstream primer for the DNA sequence of anti-human CD19SCFV : | gtcgcagctcttgggtgaactcactgtcactgacgt (SEQ ID NO.16) |
| Upstream primer for the DNA sequence of human IGHG1-Fc: | cccaagagctgcgacaagaccca (SEQ ID NO.17) |
| Downstream primer for the DNA sequence of human IGHG1-Fc: | agatcctccgccgcctttgccaggagacaggct (SEQ ID NO.18) |
| Upstream primer for the DNA sequence of human LDL-R CR2-CR3: | ggcggcggaggatctgtgacctgcaagtccgg (SEQ ID NO.19) |
| Downstream primer for the DNA sequence of human LDL-R CR2-CR3: | cggccgtcgcactcacacaggacagctggcctcgt (SEQ ID NO.20) |

The components were added to a 1.5 mL EP tube in the following proportions:

| | |
|---|---|
| MG-HSP-v1 linearized vector | 25 ng |
| CD19scfv-IgG1-Fc-LDLR-CR2/3 ligation fragment | 25 ng |
| 10× ExoIII buffer (Takara, Cat#2170A) | 1 µl |
| ddH₂O | Up to 10 µl |

The above components were mixed and allowed to stand on ice for 5 minutes. 1 µl of Exonuclease III (Takara, Cat#2170A) was added, and the mixture was gently mixed with a pipette and then allowed to stand on ice for 60 minutes. 1 µl of 0.5M EDTA (Invitrogen, Cat#11568896) was added. After mixing, the mixture was heated at 65°C for 5 minutes and then allowed to stand on ice for 5 minutes. After centrifugation, the entire mixture was added to 100 µl of DH5α, incubated on ice for 20 minutes, heat-shocked at 42°C for 45 seconds, and incubated on ice for 2 minutes. Antibiotic-free LB medium was added, and the cells were pre-shaken for 60 minutes before being plated onto plates containing ampicillin. After 16 hours, single colonies were selected for amplification and culture, and the plasmids ware extracted after confirming the correct sequence by DNA sequencing.

(2) For CD3(OKT3)-VSV-G, CD3(UCHT1)-VSV-G, CD4-VSV-G, CD20-VSV-G, CD40-VSV-G, CD45-VSV-G and HLA-VSV-G bispecific antibodies, two types of vectors were required to be constructed. Firstly, the DNA encoding the VH fragments of anti-human CD3 (OKT3), CD3 (UCHT1), CD4, CD20, CD40, CD45 or HLA was inserted before the DNA encoding the CH1-Fc fragment of the human antibody; the DNA fragment encoding CR2-CR3 of human LDL-R was inserted after the Fc fragment of the antibody; and the fully linked sequence formed by the three fragments was inserted into an expression vector that could be expressed in mammalian cells, wherein the expression vector MG-HSP.v2 (synthesized) in mammalian cells contained a CMV promoter, which can initiate the expression of downstream genes in mammalian cells. Secondly, the DNA encoding the VL fragments of anti-human CD3 (OKT3), CD3 (UCHT1), CD4, CD20, CD40, CD45 or HLA was inserted before the DNA encoding the CL fragment of the human antibody; and the fully linked sequence formed by the two fragments was inserted into an expression vector that could be expressed in mammalian cells, wherein the expression vector MG-LSP.V1 (synthesized) in mammalian cells contained a CMV promoter, which can initiate the expression of downstream genes in mammalian cells.

**Table 2**

| Fragment | Sequence (5' to 3') | Number (SEQ ID NO.) |
|---|---|---|
| DNA sequence of VH of anti-human CD3 (OKT3) | CAAGTGCAGCTCCAGCAGAGCGGCGCCGAGCTGGCCAGACCTGGAG CTTCTGTGAAAATGAGCTGCAAGGCCTCTGGCTATACCTTCACCAGAT ACACCATGCACTGGGTCAAGCAGCGGCCTGGCCAGGGCCTGGAATGG ATCGGCTACATCAACCCCAGCAGAGGCTACACCAATTACAACCAGAA ATTCAAGGACAAGGCTACACTGACAACAGATAAGAGCAGCTCCACCG CCTACATGCAGCTGAGCAGCCTGACCAGCGAGGACAGCGCCGTGTAC TACTGCGCCCGGTATTACGACGACCACTACTGTCTGGATTACTGGGGC CAGGGAACAACCCTGACCGTGTCCTCT | **SEQ ID NO.60** |
| Protein sequence of VH of anti-human CD3 (OKT3) | | **SEQ ID NO.88** |
| DNA sequence of VL of anti-human CD3 (OKT3) | CAAATCGTGCTGACACAGAGCCCTGCTATCATGAGCGCCTCTCCAGG CGAGAAGGTCACCATGACCTGTAGCGCCAGCAGCAGCGTGTCCTACA TGAACTGGTACCAGCAGAAAAGCGGCACCAGCCCCAAGCGGTGGAT CTACGACACCAGCAAGCTGGCCTCCGGCGTGCCTGCCCACTTTAGAG GCAGCGGATCTGGAACATCTTATAGCCTGACCATCAGCGGCATGGAAG CCGAGGATGCCGCTACATACTACTGCCAGCAGTGGTCCTCTAATCCTT TCACCTTCGGCAGCGGCACCAAGCTGGAAATCAACAGA | **SEQ ID NO.67** |
| Protein sequence of VL of anti-human CD3 (OKT3) | QIVLTQSPAIMSASPGEKVTMTCSASSSVSYMNWYQQKSGTSPKRWIYD TSKLASGVPAHFRGSGSGTSYSLTISGMEAEDAATYYCQQWSSNPFTFGS GTKLEINR | **SEQ ID NO.97** |
| DNA sequence of VH of anti-human CD3 (UCHTI) | GAGGTGCAGCTGCAGCAAAGCGGACCTGAGCTCGTGAAGCCTGGAG CCTCTATGAAGATCAGCTGTAAAGCCTCCGGCTACAGCTTTACCGGCT ATACAATGAACTGGGTCAAGCAGAGCCACGGCAAGAATCTGGAATGG ATGGGCCTGATCAACCCCTACAAGGGCGTGTCCACCTACAACCAGAA GTTCAAGGACAAGGCTACACTGACCGTGGACAAAAGCAGCTCTACCG CCTACATGGAACTGCTGAGCCTGACAAGCGAGGACTCCGCCGTGTAC TACTGCGCCAGAAGCGGCTACTACGGCGATTCTGATTGGTACTTCGAC GTGTGGGGCCAGGGCACCACCCTGACCGTGTTCAGC | **SEQ ID NO.61** |
| Protein sequence of VH of anti-human CD3 (UCHT1) | EVQLQQSGPELVKPGASMKISCKASGYSFTGYTMNWVKQSHGKNLEW MGLINPYKGVSTYNQKFKDKATLTVDKSSSTAYMELLSLTSEDSAVYYC ARSGYYGDSDWYFDVWGQGTTLTVFS | **SEQ ID NO.94** |
| Protein sequence of VL of anti-human CD3 (UCHT1) | ATGGACATTCAGATGACCCAGACCACCAGCAGCCTGAGCGCTTCTCT GGGCGATAGAGTGACCATCTCTTGTAGAGCCAGCCAGGACATCCGGA ACTACCTGAACTGGTATCAGCAGAAACCTGATGGCACAGTGAAGCTG CTGATCTACTACACCTCTAGACTGCACAGCGGCGTGCCCAGCAAGTTC AGCGGCTCCGGCAGCGGAACAGACTACAGCCTCACCATCTCCAACCT GGAGCAGGAGGACATCGCCACATACTTCTGCCAGCAAGGCAATACCC TGCCTTGGACCTTTGCCGGCGGAACAAAGCTGGAAATCAAG | **SEQ ID NO.68** |
| Protein sequence of VL of anti-human CD3 (UCHT1) | MDIQMTQTTSSLSASLGDRVTISCRASQDIRNYLNWYQQKPDGTVKLLI YYTSRLHSGVPSKFSGSGSGTDYSLTISNLEQEDIATYFCQQGNTLPWTF AGGTKLEIK | **SEQ ID NO.103** |
| DNA sequence of VH of anti-human CD20 | CAAGTGCAGCTGCAGCAGCCAGGAGCCGAGCTGGTGAAACCTGGAG CTAGCGTGAAGATGAGCTGTAAAGCCTCTGGCTACACCTTCACCTCTT ATAATATGCACTGGGTCAAGCAGACCCCTGGCAGAGGCCTGGAATGG ATCGGCGCTATCTACCCCGGCAACGGCGACACCAGCTACAACCAGAA GTTCAAGGGCAAGGCCACACTGACAGCCGACAAGTCCAGCTCTACA GCCTACATGCAGCTGAGCAGCCTGACCAGCGAGGACAGCGCCGTGTA CTACTGCGCCCGGAGCACCTACTACGGCGGAGATTGGTACTTTAACGT GTGGGGCGCCGGCACAACCGTGACCGTGTCCGCC | **SEQ ID NO.63** |
| Protein sequence of VH of anti-human CD20 | | **SEQ ID NO.91** |
| DNA sequence of VL of anti-human CD20 | CAAATCGTGCTGTCTCAGAGCCCTGCTATCCTGAGCGCCTCTCCAGGC GAGAAGGTGACCATGACCTGTAGAGCCAGCTCTAGCGTGTCCTATATC CACTGGTTTCAGCAGAAACCTGGCAGCAGCCCCAAGCCTTGGATCTA CGCCACCAGCAACCTGGCCAGCGGCGTCCCCGTGCGGTTCAGCGGAT CTGGCTCCGGCACCAGCTACAGCCTGACCATCAGCAGAGTGGAAGCC GAGGACGCCGCTACATACTACTGCCAGCAGTGGACCTCCAACCCTCC TACATTCGGCGGCGGAACAAAGCTGGAAATTAAG | **SEQ ID NO.70** |
| Protein sequence of VL of anti-human CD20 | | **SEQ ID NO.100** |
| DNA sequence of VH of anti-human CD40 | CAAGTGCAGCTGGTCGAGAGCGGAGGCGGCGTGGTGCAGCCTGGAA GAAGCCTGAGACTGAGCTGCGCTGCTTCTGGCTTTAGCTTCAGCTCTT ATGTGTGCTGGGTGCGGCAGGCCCCTGGCAAGGGCCTGGAATGGATC GCCTGTATCTACACCGGCGACGGCACAAATTACAGCGCCAGCTGGGC CAAGGGCAGATTCACCATCAGCAAGGACAGCTCTAAAAACACAGTGT ACCTGCAGATGAACAGCCTGCGGGCCGAGGACACCGCCGTGTACTTC TGCGCCAGACCAGATATCACCTACGGCTTCGCCATCAACTTCTGGGGC CCCGGAACACTGGTGACCGTGTCCTCC | **SEQ ID NO.64** |
| Protein sequence of VH of anti-human CD40 | QVQLVESGGGVVQPGRSLRLSCAASGFSFSSYVCWVRQAPGKGLEWIA CIYTGDGTNYSASWAKGRFTISKDSSKNTVYLQMNSLRAEDTAVYFCAR PDITYGFAINFWGPGTLVTVSS | **SEQ ID NO.92** |
| DNA sequence of VL of anti-human CD40 | ATGGACATGCGGGTCCCCGCCCAGCTGCTCGGCCTGCTGCTGCTGTG GCTGCGCGGCGCTAGATGCGACATCCAGATGACCCAGTCCCCTAGCA GCCTGAGCGCCAGCGTGGGCGATAGAGTGACCATCAAGTGCCAGGCC AGCCAGAGCATCTCCTCCAGACTGGCTTGGTATCAGCAGAAGCCCGG CAAGCCTCCTAAGCTGCTGATCTACCGGGCCTCTACACTGGCCTCTGG CGTGCCATCTAGATTCAGCGGCAGCGGATCTGGAACAGATTTCACCCT GACCATTAGCAGCCTGCAGCCTGAGGACGTGGCCACATACTACTGCC AATGTACCGGCTACGGCATCAGCTGGCCTATCGGCGGCGGAACCAAG GTGGAAATCAAA | **SEQ ID NO.71** |
| Protein sequence of VL of anti-human CD40 | MDMRVPAQLLGLLLLWLRGARCDIQMTQSPSSLSASVGDRVTIKCQASQ SISSRLAWYQQKPGKPPKLLIYRASTLASGVPSRFSGSGSGTDFTLTISSL QPEDVATYYCQCTGYGISWPIGGGTKVEIK | **SEQ ID NO.101** |
| DNA sequence of VH of anti-human CD4 | GAGGTGCAACTGGTGGAAAGCGGCGGAGGACTGGTGCAGCCCGGCC GGAGCCTGCGGCTGAGCTGTGCCGCTTCTGGTTTTACCTTCAGCAACT ACGGCATGGCCTGGGTCAGACAGGCCCCTGGCAAAGGCCTCGAGTG GGTGGCTACAATCAGCTACGACGGCAGCATCACCTACTATAGAGATAG CGTGAAGGGCAGATTCACAATCTCTAGAGATAATAGCAAGAACACCC TGTACCTGCAGATGAACTCTCTGCGGGCCGAGGACACCGCCGTGTAC TACTGCACCAGAGAGGAACAGTACAGCAGCTGGTACTTCGACTTCTG GGGCCAGGGCATCCTGGTTACAGTGTCCTCC | **SEQ ID NO.62** |
| Protein sequence of VH of anti-human CD4 | EVQLVESGGGLVQPGRSLRLSCAASGFTFSNYGMAWVRQAPGKGLEW VATISYDGSITYYRDSVKGRFTISRDNSKNTLYLQMNSLRAEDTAVYYCT REEQYSSWYFDFWGQGILVTVSS | **SEQ ID NO.93** |
| DNA sequence of VL of anti-human CD4 | GATATCCAGCTGACACAGTCTCCATCTAGCCTGAGCGCCTCTGTGGGC GATAGAGTGACAATCACCTGTAGAGCCTCCCAGAGCGTGTCCATCAG CAGCCACGACCTGATGCAGTGGTATCAGCAGAAACCTGGCAAGGCCC CTAAGCTCCTGATCTACGACGCCTTCAACCTGGCCAGCGGCGTCCCCA GCCGGTTCAGCGGATCTGGCAGCGGAACCGACTTCACCCTGACCATT AGCTCCCTGCAACCTGAGGATTTTGCTACATACTACTGCCAGCAAAGC AAGGACGACCCCTACACCTTCGGCCAGGGCACCAAGCTGGAAATCA AG | **SEQ ID NO.69** |
| Protein sequence of VL of anti-human CD4 | DIQLTQSPSSLSASVGDRVTITCRASQSVSISSHDLMQWYQQKPGKAPKL LIYDAFNLASGVPSRFSGSGSGTDFTLTISSLQPEDFATYYCQQSKDDPYT FGQGTKLEIK | **SEQ ID NO.102** |
| DNA sequence of human CD40 ligand | GGTGATCAGAATCCTCAAATTGCGGCACATGTCATAAGTGAGGCCAG CAGTAAAACAACATCTGTGTTACAGTGGGCTGAAAAAGGATACTACA CCATGAGCAACAACTTGGTAACCCTGGAAAATGGGAAACAGCTGACC GTTAAAAGACAAGGACTCTATTATATCTATGCCCAAGTCACCTTCTGTT CCAATCGGGAAGCTTCGAGTCAAGCTCCATTTATAGCCAGCCTCTGCC TAAAGTCCCCCGGTAGATTCGAGAGAATCTTACTCAGAGCTGCAAATA CCCACAGTTCCGCCAAACCTTGCGGGCAACAATCCATTCACTTGGGA GGAGTATTTGAATTGCAACCAGGTGCTTCGGTGTTTGTCAATGTGACT GATCCAAGCCAAGTGAGCCATGGCACTGGCTTCACGTCCTTTGGCTTA CTCAAACTC | **SEQ ID NO.105** |
| Protein sequence of human CD40 ligand | GDQNPQIAAHVISEASSKTTSVLQWAEKGYYTMSNNLVTLENGKQLTV KRQGLYYIYAQVTFCSNREASSQAPFIASLCLKSPGRFERILLRAANTHSS AKPCGQQSIHLGGVFELQPGASVFVNVTDPSQVSHGTGFTSFGLLKL | **SEQ ID NO.106** |
| DNA sequence of VH of anti-human HLA | CAGGTGCAGCTGAAACAGAGCGGACCTGGCCTGGTCCAGCCTAGCC AGTCCCTGAGCCTGACATGCACCGTGTCCGGCTTCAGCCTGACCAGC TACGGCGTGCACTGGGTGCGGCAGCCTCCCGGCAAGGGCCTCGAGTG GCTGGGCGTGATCTGGTCCGGCGGATCTACAGATTACAACGCCGCTTT TATCAGCAGACTGAGCATCAGAAAGGACAATAGCAAGAGCCAAGTGT TCTTCAAGATGAACAGCCTGCAGGCCGACGACACCGCCATCTACTAC TGCGCCAGAACCTTCACCACAAGCACCAGCGCCTGGTTCGCCTATTG GGGCCAGGGCACCCTGGTGACCGTGTCTGCT | **SEQ ID NO.66** |
| Protein sequence of VH of anti-human HLA | QVQLKQSGPGLVQPSQSLSLTCTVSGFSLTSYGVHWVRQPPGKGLEWLG VIWSGGSTDYNAAFISRLSIRKDNSKSQVFFKMNSLQADDTAIYYCARTF TTSTSAWFAYWGQGTLVTVSA | **SEQ ID NO.95** |
| DNA sequence of VL of anti-human HLA | TCTATCGTGATGACCCAAACACCTAAGTTCCTGCTGGTCTCCGCCGGA GATAGAGTGACCATCACCTGCAAGGCTTCTCAGAGCGTGTCCAACGA CGTGGCCTGGTATCAGCAAAAGCCCGGCCAGAGCCCCAAGCTGCTGA TCTACTACGCCAGCAACCGGTACACCGGCGTGCCTGATAGATTCACAG GCAGCGGCTACGGCACCGACTTCACATTTACCATCAGCACCGTGCAG GCCGAGGACCTGGCCGTGTACTTCTGCCAGCAGGACTACAGCAGCCC TCCATGGACCTTCGGCGGAGGCACAAAGCTGGAAATCAGA | **SEQ ID NO.73** |
| Protein sequence of VL of anti-human HLA | SIVMTQTPKFLLVSAGDRVTITCKASQSVSNDVAWYQQKPGQSPKLLIYY ASNRYTGVPDRFTGSGYGTDFTFTISTVQAEDLAVYFCQQDYSSPPWTF GGGTKLEIR | **SEQ ID NO.104** |
| DNA sequence of VH of anti-human CD45 | CAAGTGCAACTGGTGGAATCTGGAGGAGGACTGGTGCAGCCCGGCG GCAGCCTGAAACTGAGCTGCGCCGCCAGCGGCTTCGACTTTTCTAGG TACTGGATGAGCTGGGTGCGGCAGGCCCCTGGCAAGGGCCTGGAATG GATCGGCGAGATCAATCCTACAAGCAGCACCATCAACTTCACCCCTAG TCTGAAGGACAAGGTGTTCATCAGCCGCGACAACGCCAAGAACACA CTGTACCTGCAGATGAGCAAGGTGCGGAGCGAGGACACCGCCCTCTA CTACTGCGCCAGAGGCAACTACTACAGATACGGCGACGCCATGGACT ACTGGGGCCAGGGCACAAGCGTGACAGTGTCCAAAATCAGC | **SEQ ID NO.65** |
| Protein sequence of VH of anti-human CD45 | QVQLVESGGGLVQPGGSLKLSCAASGFDFSRYWMSWVRQAPGKGLEWI GEINPTSSTINFTPSLKDKVFISRDNAKNTLYLQMSKVRSEDTALYYCAR GNYYRYGDAMDYWGQGTSVTVSKIS | **SEQ ID NO.107** |
| DNA sequence of VL of anti-human CD45 | AGCGACATCGTGCTGACCCAGAGCCCTGCTTCTCTGGCCGTGTCTCTG GGCCAGCGGGCCACAATTAGCTGTAGAGCCAGCAAGTCCGTGTCCAC CAGCGGTTATTCTTACCTGCACTGGTACCAGCAGAAGCCTGGCCAGC CACCTAAGCTGCTGATCTACCTGGCCAGCAACCTGGAATCTGGCGTCC CCGCTAGATTCTCTGGAAGCGGCAGTGGCACCGATTTCACCCTGAAC ATCCACCCCGTGGAGGAAGAAGATGCCGCTACCTACTATTGCCAGCA CAGCAGAGAGCTGCCATTCACATTTGGCAGCGGCACCAAGCTCGAGA TCAAG | **SEQ ID NO.72** |
| Protein sequence of VL of anti-human CD45 | SDIVLTQSPASLAVSLGQRATISCRASKSVSTSGYSYLHWYQQKPGQPPK LLIYLASNLESGVPARFSGSGSGTDFTLNIHPVEEEDAATYYCQHSRELPF TFGSGTKLEIK | **SEQ ID NO.108** |

The VH and VL fragments of anti-human CD3 (OKT3) were obtained by optimizing the DNA sequences according to the article (DOI: 10. 1073/pnas.0402295 101) using the codon optimization tool of GenScript (https://www.genscript.com/gensmart-free-gene-codon- optimization.html) and then synthesizing them (wherein the VH DNA sequence is shown as SEQ ID NO. 60 and the VL DNA sequence is shown as SEQ ID NO. 67); the VH and VL fragments of anti-human CD3 (UCHT1) were obtained by optimizing the DNA sequences according to the article (DOI: 10.1073/pnas.0407359101) using the codon optimization tool of GenScript (https://www.genscript.com/gensmart-free-gene-codon-optimization.html) and then synthesizing them (wherein the VH DNA sequence is shown as SEQ ID NO. 61 and the VL DNA sequence is shown as SEQ ID NO. 68); the VH and VL fragments of anti-human CD4 were obtained by optimizing the DNA sequences according to the patent (https://patents.google.com/patent/CN101245108B/en) using the codon optimization tool of GenScript (https://www.genscript.com/gensmart-free-gene-codon-optimization.html) and then synthesizing them (wherein the VH DNA sequence is shown as SEQ ID NO. 62 and the VL DNA sequence is shown as SEQ ID NO. 69); the VH and VL fragments of anti-human CD20 were obtained by optimizing the DNA sequences according to the patent (https://patents.google.com/patent/CN101245108B/en) using the codon optimization tool of GenScript (https://www.genscript.com/gensmart-free-gene-codon-optimization.html) and then synthesizing them (wherein the VH DNA sequence is shown as SEQ ID NO. 63 and the VL DNA sequence is shown as SEQ ID NO. 70); the VH and VL fragments of anti-human CD40 were obtained by optimizing the DNA sequences according to the patent (https://patents.google.com/patent/EP3925977A1/en) using the codon optimization tool of GenScript (https://www.genscript.com/gensmart-free-gene-codon-optimization.html) and then synthesizing them (wherein the VH DNA sequence is shown as SEQ ID NO. 64 and the VL DNA sequence is shown as SEQ ID NO. 71); the VH and VL fragments of anti-human CD45 were obtained by optimizing the DNA sequences according to the database (GenBank: QES69311.1) using the codon optimization tool of GenScript (https://www.genscript.com/gensmart-free-gene-codon-optimization.html) and then synthesizing them (wherein the VH DNA sequence is shown as SEQ ID NO. 65 and the VL DNA sequence is shown as SEQ ID NO. 72); the VH and VL fragments of anti-human HLA were obtained by optimizing the DNA sequences according to the article (DOI: DOI: 10.1016/0092-8674(78)90296-9) using the codon optimization tool of GenScript (https://www.genscript.com/gensmart-free-gene-codon-optimization.html) and then synthesizing them (wherein the VH DNA sequence is shown as SEQ ID NO. 66 and the VL DNA sequence is shown as SEQ ID NO. 73); the DNA encoding the CH1-Fc fragment of the human antibody was obtained by optimizing the DNA sequence according to the protein database UniProt (P01857·IGHG1_HUMAN) using the codon optimization tool of GenScript (https://www.genscript.com /gensmart-free-gene-codon-optimization.html) and then synthesizing it (whose nucleotide sequence is shown as SEQ ID NO. 74), the DNA encoding the CL fragment of the human antibody was obtained by optimizing the DNA sequence according to the protein database UniProt (P01834·IGKC_HUMAN) using the codon optimization tool of GenScript (https://www.genscript.com /gensmart-free-gene-codon-optimization.html) and then synthesizing it (whose nucleotide sequence is shown as SEQ ID NO. 75), and the DNA encoding the human LDL-R CR2-CR3 was the same as that in (1).

The required fragments were ligated together using overlap extension polymerase chain reaction. The primers used are shown in Table 3 below. The expression vector MG-HSP-v2 (whose nucleotide sequence is shown as SEQ ID NO. 59) or MG-LSP-v1 (whose nucleotide sequence is shown as SEQ ID NO. 76) was treated with the BsaI restriction endonuclease (NEB, Cat# R3733L) to linearize it.

**Table 3**

| Primer name | Sequence (5' to 3') |
|---|---|
| Upstream primer for the DNA sequence of anti-human CD3: | **acaggtgcccattctcaagtgcagctccagcagag** (SEQ ID NO.21) |
| Downstream primer for the DNA sequence of anti-human CD3: | **acccttggtgctggcagaggacacggtcagggttg** (SEQ ID NO.22) |
| Upstream primer for the DNA sequence of human IGHG1-CH1-Fc: | **gccagcaccaagggtcccag** (SEQ ID NO.23) |
| Downstream primer for the DNA sequence of human IGHG1-CH1-Fc : | **agatcctccgccgccctttccggggctcaggctca** (SEQ ID NO.24) |
| Upstream primer for the DNA sequence of human LDL-R CR2-CR3: | **ggcggcggaggatctgtgacctgenagtccgg** (SEQ ID NO.25) |
| Downstream primer for the DNA sequence of human LDL-R CR2-CR3: | **gatcgaaccctttcatcacacageacagctggcctcgt** (SEQ ID NO.26) |
| Upstream primer for the DNA sequence of VL of anti-human CD3: | **ccaggctctaccggccaaatcgtgctgacacagag** (SEQ ID NO.27) |
| Downstream primer for the DNA sequence of VL of anti-human CD3: | **ggcagccacggtccttctgttgatttccagcttgg** (SEQ ID NO.28) |
| Upstream primer for the DNA sequence of human IGKC-CL: | **aggaccgtggctgcccccag** (SEQ ID NO.29) |
| Downstream primer for the DNA sequence of human IGKC-CL : | **gatcgaaccctttcatcagcactcgcccctgttgaagc** (SEQ ID NO.30) |
| Upstream primer for the DNA sequence of VH of anti-human CD45: | **acaggtgcccattctcaagtgcaactggtggaatc** (SEQ ID NO.31) |
| Downstream primer for the DNA sequence of VH of anti-human CD45: | **acccttggtgctggcgctgattttggacactgtca** (SEQ ID NO.32) |
| Upstream primer for the DNA sequence of VL of anti-human CD45: | **ccaggctctaccggcagcgacatcgtgctgaccca** (SEQ ID NO.33) |
| Downstream primer for the DNA sequence of VL of anti-human CD45: | **ggcagccacggtcctcttgatctcgagcttggtgc** (SEQ ID NO.34) |
| Upstream primer for the DNA sequence of VH of anti-human CD3(UCHT I): | **acaggtgcccattctgaggtgcagctgcagcaaag** (SEQ ID NO.35) |
| Downstream primer for the DNA sequence of VH of anti-human CD3(UCHTI): | **acccttggtgctggcgctgaacacggtcagggtgg** (SEQ ID NO.36) |
| Upstream primer for the DNA sequence of VL of anti-human CD3(UCHTI1): | **ccaggctctaccggcatggacattcagatgaccca** (SEQ ID NO.37) |
| Downstream primer for the DNA sequence of VL of anti-human CD3(UCHTI): | **ggcagccacggtcctcttgatttccagctttgttcc** (SEQ ID NO.38) |
| Upstream primer for the DNA sequence of VH of anti-human CD4: | **acaggtgcccattctgaggtgcaactggtggaaag** (SEQ ID NO.39) |
| Downstream primer for the DNA sequence of VH of anti-human CD4: | **acccttggtgctggcggaggacactgtaaccagga** (SEQ ID NO.40) |
| Upstream primer for the DNA sequence of VL of anti-human CD4: | **ccaggctctaccggcgatatccagctgacacagtct** (SEQ ID NO.41) |
| Downstream primer for the DNA sequence of VL of anti-human CD4: | **ggcagccacggtcctcttgatttccagcttggtgc** (SEQ ID NO.42) |
| Upstream primer for the DNA sequence of VH of anti-human CD20: | **acaggtgcccattctcaagtgcagctgcagcagccagga** (SEQ ID NO.43) |
| Downstream primer for the DNA sequence of VH of anti-human CD20: | **acccttggtgctggcggcggacacggtcacggttg** (SEQ ID NO.44) |
| Upstream primer for the DNA sequence of VL of anti-human CD20: | **ccaggctctaccggccaaatcgtgctgtctcagag** (SEQ ID NO.45) |
| Downstream primer for the DNA sequence of VL of anti-human CD20: | **ggcagccacggtcctcttaatttccagctttgttcc** (SEQ ID NO.46) |
| Upstream primer for the DNA sequence of VH of anti-human CD40: | **acaggtgccccattctcaagtgcagctggtcgagag** (SEQ ID NO.47) |
| Downstream primer for the DNA sequence of VH of anti-human CD40: | **acccttggtgctggcggaggacacggtcaccagtg** (SEQ ID NO.48) |
| Upstream primer for the DNA sequence of VL of anti-human CD40: | **ccaggctctaccggcatggacatgcgggtccccgccca** (SEQ ID NO.49) |
| Downstream primer for the DNA sequence of VL of anti-human CD40: | **ggcagccacggtccttttgatttccaccttggttcc** (SEQ ID NO.50) |
| Upstream primer for the DNA sequence of VH of anti-human HLA: | **acaggtgcccattctcaggtgcagctgaaacagag** (SEQ ID NO.51) |
| Downstream primer for the DNA sequence of VH of anti-human HLA: | **accettggtgctggcagcagacacggtcaccagggt** (SEQ ID NO.52) |
| Upstream primer for the DNA sequence of VL of anti-human HLA: | **ccaggctctaccggctctatcgtgatgacccaaacacct** (SEQ ID NO.53) |
| Downstream primer for the DNA sequence of VL of anti-human HLA: | **ggcagccacggtccttctgatttccagctttgtgcctcc** (SEQ ID NO.54) |

For CD3(OKT3) (or CD3(UCHT1), CD4, CD20, CD40, CD45, HLA)-VH-IgG1-CH1-Fc-LDLR-CR2/3 plasmid, the components were added to a 1.5 mL Ep tube in the following proportions:

| | |
|---|---|
| MG-HSP-v2 linearized vector | 25 ng |
| CD3(OKT3) (or CD3(UCHT1), CD4, CD20, CD40, CD45, HLA)-VH-IgG1-CH1-Fc-LDLR-CR2/3 ligation fragment | 25 ng |
| 10× ExoIII buffer (Takara, Cat#2170A) | 1 µL |
| ddH₂O | Up to 10 µL |

For CD3(OKT3) (or CD3(UCHT1), CD4, CD20, CD40, CD45, HLA)-VL-CL plasmid, the components were added to a 1.5 mL Ep tube in the following proportions:

| | |
|---|---|
| MG-LSP-v1 linearized vector | 25 ng |
| CD3(OKT3) (or CD3(UCHT1), CD4, CD20, CD40, CD45, HLA)-VL-CL ligation fragment | 25 ng |
| 10× ExoIII buffer (Takara, Cat#2170A) | 1 µL |
| ddH₂O | Up to 10 µL |

The above components were mixed and allowed to stand on ice for 5 minutes. 1 µL of Exonuclease III (Takara, Cat#2170A) was added, and the mixture was gently mixed with a pipette and then allowed to stand on ice for 60 minutes. 1 µL of 0.5M EDTA (Invitrogen, Cat#11568896) was added. After mixing, the mixture was heated at 65°C for 5 minutes and then allowed to stand on ice for 5 minutes. After centrifugation, the entire mixture was added to 100 µL of DH5α, incubated on ice for 20 minutes, heat-shocked at 42°C for 45 seconds, and incubated on ice for 2 minutes. Antibiotic-free LB medium was added, and the cells were pre-shaken for 60 minutes before being plated onto plates containing ampicillin. After 16 hours, single colonies were selected for amplification and culture, and the plasmids ware extracted after confirming the correct sequence by DNA sequencing.

(3) For CD40L-VSV-G bispecific antibodies, the vector construction involved inserting the DNA fragment encoding CR2-CR3 of human LDL-R before the Fc fragment of the human antibody, inserting the DNA fragment encoding the human CD40 ligand fragment after the DNA encoding the Fc fragment of the human antibody, and inserting the fully linked sequence of the three fragments into an expression vector that could be expressed in mammalian cells, wherein the expression vector MG-HSP-v1 in mammalian cells contains a CMV promoter, which can initiate the expression of downstream genes in mammalian cells.

The human CD40 ligand fragment was obtained by optimizing the DNA sequences according to the article (https://www.genscript.com/gensmart-free-gene-codon-optimization.html) using the UniProt protein database (P29965 · CD40L_HUMAN) and then synthesizing them. Its nucleotide sequence is shown as SEQ ID NO.; the DNA encoding the Fc fragment of the human antibody was the same as that in (1), and the DNA encoding the human LDL-R CR2-CR3 was the same as that in (1).

The three fragments were linked together using overlap extension polymerase chain reaction. The primers used are shown in Table 1 below. The expression vector MG-HSP-v1 (whose nucleotide sequence is shown as SEQ ID NO. 55) was treated with BsaI restriction endonuclease (NEB, Cat# R3733L) to linearize it.

**Table 4**

| Primer name | Sequence (5' to 3') |
|---|---|
| Upstream primer for the DNA sequence of human LDL-R CR2-CR3: | **accggtgtacattctgtgacctgcaagtccggcga** (SEQ ID NO.77) |
| Downstream primer for the DNA sequence of human LDL-R CR2-CR3: | **cggatccaccacctcccacaggacagctggcctc** (SEQ ID NO.78) |
| Upstream primer for the DNA sequence of human IGHG1-Fc: | **ggaggtggtggatccgagcccaaatcttgtgacaaaac** (SEQ ID NO.79) |
| Downstream primer for the DNA sequence of human IGHGI-Fc: | **tccagagcctttacccggggacagggaga** (SEQ ID NO.80) |
| Upstream primer for the DNA sequence of human CD40 ligand: | **ggtaaaggctctggaggtgatcagaatcctcaaattgc** (SEQ ID NO.81) |
| , Downstream primer for the DNA sequence of human CD40 ligand: | **cggccgtcgcactcagagtttaagtaagccaaagg** (SEQ ID NO.82) |

The components were added to a 1.5 mL Ep tube in the following proportions:

| | |
|---|---|
| MG-LSP-v1 linearized vector | 25 ng |
| LDLR-CR2/3-IgG1-Fc-CD40L ligation fragment | 25 ng |
| 10× ExoIII buffer (Takara, Cat#2170A) | 1 µL |
| ddH₂O | Up to 10 µL |

The above components were mixed and allowed to stand on ice for 5 minutes. 1 µL of Exonuclease III (Takara, Cat#2170A) was added, and the mixture was gently mixed with a pipette and then allowed to stand on ice for 60 minutes. 1 µL of 0.5M EDTA (Invitrogen, Cat#11568896) was added. After mixing, the mixture was heated at 65°C for 5 minutes and then allowed to stand on ice for 5 minutes. After centrifugation, the entire mixture was added to 100 µL of DH5α, incubated on ice for 20 minutes, heat-shocked at 42°C for 45 seconds, and incubated on ice for 2 minutes. Antibiotic-free LB medium was added, and the cells were pre-shaken for 60 minutes before being plated onto plates containing ampicillin. After 16 hours, single colonies were selected for amplification and culture, and the plasmids ware extracted after confirming the correct sequence by DNA sequencing.

### Example 1: Preparation of Bispecific Antibody Molecules

(1) Day -2: Expi-293F cells were cultured in suspension in Union293 cell culture medium (adding additional 1% GlutaMax) in a 37°C incubator with 5% CO₂ at a rotation speed of 180 rpm. The freshly thawed cells were ensured that had been cultured for three or more generations and exhibited a viability of ≥90%. The Epi293F cells were cultured and amplified until the day before transfection (Day -1), when the cell density should reach approximately 3-5×10⁶ cells/mL.
(2) Day -1: Cells were inoculated at a final density of 2.5-3×10⁶ cells/mL and allowed to grow overnight.
(3) Day 0: The living cell density and viability were determined. Transfection was performed only if the density of the living cells reached 4.5-5.5×10⁶ cells/mL and viability reached ≥95%, respectively. Cells were diluted to 3×10⁶ living cells/mL using pre-warmed Union293 cell culture medium (supplemented with 1% GlutaMax), and the culture flask was gently shaken to mix the cells. The mixture for transfection was prepared in a sterile 15 mL centrifuge tube (using a 30 mL expression system as an example):

| | |
|---|---|
| Opti-MEM reduced serum medium | 3 mL |
| Total expression plasmids (light chain: heavy chain = 2.5:1) | 24 µg |
| Polyethylenimine (PEI) transfection reagent | 96 µg |

The mixture was gently mixed using a pipette and left to stand at room temperature for 10 minutes. 27 mL of Expi-293F cells at a concentration of 3×10⁶/mL were added to a disposable sterile vented shake flask, and the mixture was added dropwise to the cells in the culture dish and gently shaken to mix evenly. The cells were incubated in a 37°C incubator with 5% CO₂ for 24 hours.
(4) Day 1: 300 µL of 300 mM valproic acid (VPA, Sigma cat # P4543; prepared with water and filtered through a 0.22 µm filter) and 270 µL of 45% glucose (Sigma cat# G8769) were added to the cells, gently shaking to mix. The cells were incubated in a 37°C incubator with 5% CO.
(5) Day 5: The protein was harvested. The cells were transferred to 50 mL centrifuge tubes and centrifuged at 4,000 rpm for 10 minutes at 4°C. The supernatant was transferred to new 50 mL centrifuge tubes.
(6) 400 µL of Protein G purification medium (Genscript cat# L00664) was taken after mixing, added with 1 mL of 1×PBS, centrifuged at 600g for 10 minutes at 4°C to discard the supernatant. The washing procedure was repeated for three times to remove ethanol from the storage solution. The washed Protein G purification medium was added to the supernatant obtained in step (5), and incubated with rotation at 4°C for 2-4 hours.
(7) A gravity column was prepared for purification. A hydrophilic sieve plate of appropriate size which was pre-wetted using equilibration/wash buffer (25 mM Tris, 150 mM NaCl; pH 7.2) was placed into a 3 mL empty affinity chromatography column tube. The incubated supernatant was transferred to the column tube, allowing unbound proteins and impurities to flow out with the supernatant of culture medium by gravity while the Protein G purification medium bound to the target proteins was accumulated in the column tube under the effect of the sieve plate. The Protein G purification medium was washed by slowly adding 10 times the column volume of equilibration/wash buffer to the column tube. 1 mL of eluent (0.1 M glycine, pH 2-3) was slowly added, and the eluate was collected at the outlet (pre-filled with 1/10 volume of neutralization buffer (1 M Tris, pH 7.5-9)). The protein concentration was measured, and the elution step was repeated until the protein concentration in the outflow was undetectable.
(8) The protein solvent was replaced from the eluent with 1×PBS using 50 kDa ultrafiltration tubes, concentrated to 0.5 mg/mL, filtered through a 0.22 µm filter, aliquoted, and stored at 4°C for short-term storage or at -20°C for extended storage.

Among them, the heavy chain sequence of the CD3-VSV-G bispecific antibody is shown as SEQ ID NO.2, and the light chain sequence is shown as SEQ ID NO.3; the heavy chain sequence of the CD45-VSV-G bispecific antibody is shown as SEQ ID NO.4, and the light chain sequence is shown as SEQ ID NO.5; the sequence of the CD19scfv-VSV-G bispecific antibody is shown as SEQ ID NO.6.

### Example 2: Packaging of VSV-G Pseudotyped Lentiviruses

(1) Day 0: HEK293T cells were digested with trypsin, and a certain number of cells was collected and plated in six-well plates. The number of cells should be controlled to reach approximately 70-80% confluence by the time of transfection on the next day to enhance transfection efficiency. The cells were placed in a 37°C incubator with 5% CO₂ for culture for 8-24 hours.
(2) Day 1: Transfection was started once the cells were completely attached and reached an appropriate density. Two hours before transfection, the HEK293T cell culture medium was aspirated using a vacuum pump and replaced with 1.5 mL of fresh DMEM medium (the medium was supplemented with 10% FBS (inactivated by heating at 56°C for 30 minutes), 1% penicillin-streptomycin, referred to as DMEM complete medium hereinafter). The plasmid mixture for transfection was prepared in sterile 1.5 mL Eppendorf tubes according to the following formula for each reaction:

| | |
|---|---|
| Opti-MEM reduced serum medium | 400 µL |
| pBOB-GFP (synthetic, SEQ ID NO.83) | 2 µg |
| pRev packaging plasmid (synthetic, SEQ ID NO.85) | 0.67 µg |
| pMDL packaging plasmid (synthetic, SEQ ID NO.86) | 0.67 µg |
| pVSV-G packaging plasmid (synthetic, SEQ ID NO.84) | 0.67 µg |
| polyethylenimine (PEI) transfection reagent | 8 µg |

The mixture was gently mixed using a pipette and left to stand at room temperature for 10 minutes. After standing, the mixture was added dropwise to the cells in the culture dish and gently shaken in a "cross" or "8" shape to mix evenly. After the cells were placed in a 37°C incubator with 5% CO₂ for culture for 8 hours, 2 mL of fresh complete DMEM medium was replaced with.

(3) Day 3: 48 hours after transfection, the culture medium of HEK293T cells was collected in sterile Eppendorf tubes. The culture medium was centrifuged at 500 g for 10 minutes at 4°C to remove cell debris and impurities, and then filtered using syringes and filters to obtain VSV-G pseudotyped lentiviruses carrying mRNA of GFP fluorescent proteins. The filtered virus solution was aliquoted and stored at -80°C for later use.

### Example 3: Enhancement of Lentiviral Infection of Ramos Cells Using Bispecific Molecules (Targeting HLA)

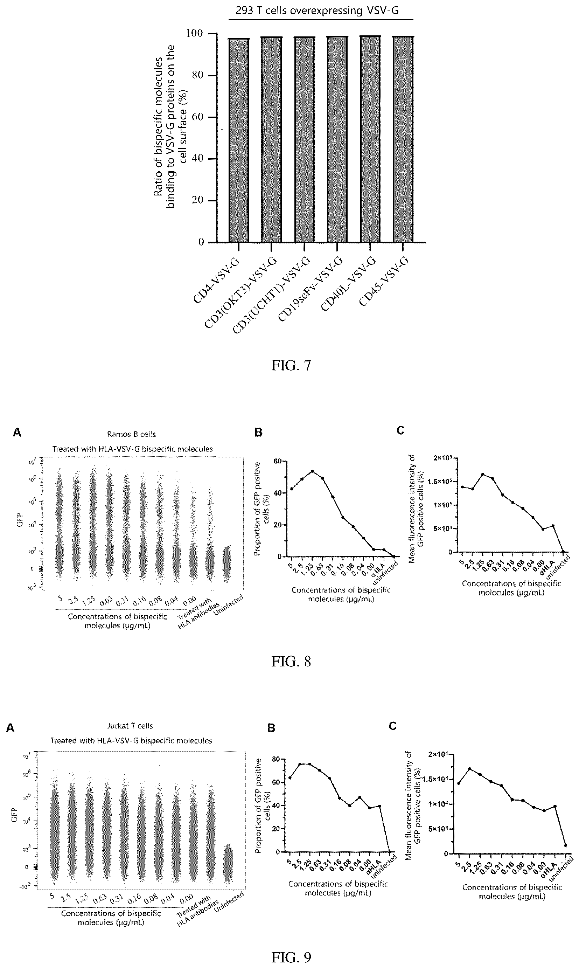

(1) Day 1: Target cells (Ramos cells) were cultured in IMDM medium (the medium was supplemented with 10% FBS (inactivated by heating at 56°C for 30 minutes), 1% penicillin-streptomycin, and 1% GlutaMAX, referred to as IMDM complete medium hereinafter). Before infection, the target cells were collected and counted. A specific number of cells was collected into sterile 1.5 mL Eppendorf tubes (approximately 5×10⁵ cells per infection reaction) and centrifuged at 300 g for 3 minutes at 4°C. The medium was discarded.
(2) The HLA-VSV-G bispecific molecules were serially diluted with pre-chilled IMDM medium (supplemented with 2% FBS) to achieve final concentrations of 5, 2.5, 1.25, 0.63, 0.31, 0.16, 0.08, 0.04, and 0.00 µg/mL, respectively (meanwhile, 2.5 µg/mL of antibodies targeting only HLA (without the LDLR-CR2-CR3 domain) was used as a control). The centrifuged cells from step (1) were resuspended with 50 µL of the mixture and incubated in a 4°C refrigerator for 15 minutes. After incubation, 1 mL of pre-chilled IMDM medium (supplemented with 2% FBS) was added to the cell suspension to dilute the concentration of bispecific molecules in the system. The mixture was centrifuged at 300 g for 3 minutes at 4°C. The medium was discarded.
(3) The cells were resuspended again with 1 mL of pre-chilled IMDM medium (supplemented with 2% FBS) and centrifuged at 300 g for 3 minutes at 4°C. The medium was discarded. This step was repeated once more.
(4) The mixture of VSV-G pseudotyped lentiviruses for infection was prepared. The viruses were diluted to an MOI of approximately 0.7 using pre-chilled IMDM complete medium. The cells collected by centrifugation in step (3) (with bispecific molecules bound to their surface at this time) were resuspended with 0.5 mL of the virus mixture, gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 300 g for 3 minutes at room temperature. The medium was discarded. The cells were resuspended with 1 mL of IMDM complete medium pre-warmed to 37°C and inoculated into 24-well plates. The cell status and density were checked daily. If necessary, the medium was replaced with fresh medium or the cell culture volume was expanded to ensure that the cell density did not exceed 3×10⁶ cells/mL.
(7) Day 4: 60 hours after infection, the infection efficiency was detected using a flow cytometer, with uninfected cells used as a control. The results are shown in FIGS. 8A to 8C.

### Example 4: Enhancement of Lentiviral Infection of Jurkat T Cells Using Bispecific Molecules (Targeting HLA)

(1) Day 1: Target cells (Jurkat T cells) were cultured in RPMI-1640 medium (the medium was supplemented with 10% FBS (inactivated by heating at 56°C for 30 minutes), 1% penicillin-streptomycin, 1% GlutaMAX, 0.1% 2-mercaptoethanol (55 mM), referred to as RPMI-1640 complete medium hereinafter). Before infection, the target cells were collected and counted. A specific number of cells was collected into sterile 1.5 mL Eppendorf tubes (approximately 5×10⁵ cells per infection reaction), and centrifuged at 300 g for 3 minutes at 4°C. The medium was discarded.
(2) The HLA-VSV-G bispecific molecules were serially diluted with pre-chilled RPMI-1640 medium (supplemented with 2% FBS) to achieve final concentrations of 5, 2.5, 1.25, 0.63, 0.31, 0.16, 0.08, 0.04, and 0.00 µg/mL, respectively (meanwhile, 2.5 µg/mL of antibodies targeting only HLA (without the LDLR-CR2-CR3 domain) was used as a control). The centrifuged cells from step (1) were resuspended with 50 µL of the mixture and incubated in a 4°C refrigerator for 15 minutes. After incubation, 1 mL of pre-chilled RPMI-1640 medium (supplemented with 2% FBS) was added to the cell suspension to dilute the concentration of bispecific molecules in the system. The mixture was centrifuged at 300 g for 3 minutes at 4°C. The medium was discarded.
(3) The cells were resuspended again with 1 mL of pre-chilled RPMI-1640 medium (supplemented with 2% FBS) and centrifuged at 300 g for 3 minutes at 4°C. The medium was discarded. This step was repeated once more.
(4) The mixture of VSV-G pseudotyped lentiviruses for infection was prepared. The viruses were diluted to an MOI of approximately 1 using pre-chilled RPMI-1640 complete medium. The cells collected by centrifugation in step (3) (with bispecific molecules bound to their surface at this time) were resuspended with 0.5 mL of the virus mixture, gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 300 g for 3 minutes at room temperature. The medium was discarded. The cells were resuspended with 1 mL of RPMI-1640 complete medium pre-warmed to 37°C and inoculated into 24-well plates. The cell status and density were checked daily. If necessary, the medium was replaced with fresh medium or the cell culture volume was expanded to ensure that the cell density did not exceed 3×10⁶ cells/mL.
(6) Day 4: 60 hours after infection, the infection efficiency was detected using a flow cytometer, with uninfected cells used as a control. The results are shown in FIGS. 9A to 9C.

### Example 5: Enhancement of Lentiviral Infection of Human Peripheral Blood Mononuclear Cells Using Bispecific Molecules (Targeting HLA)

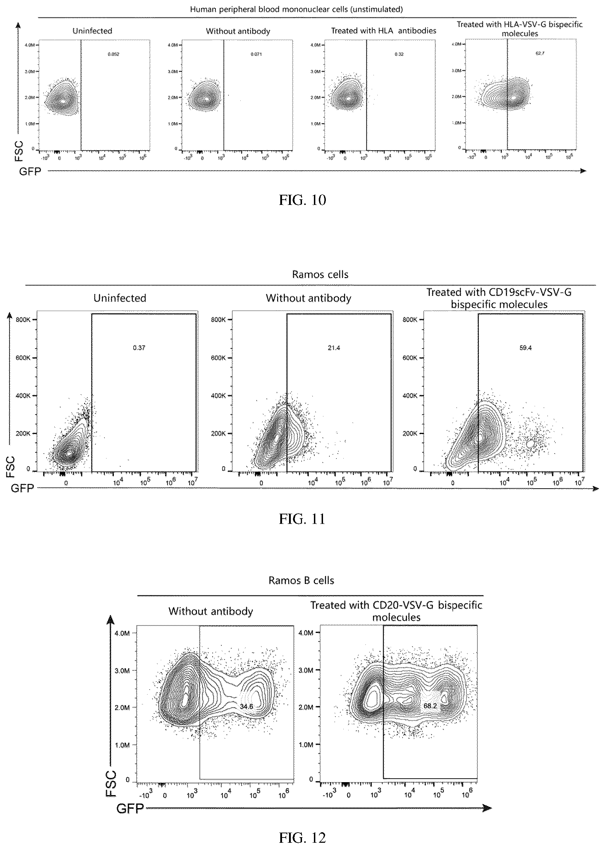

(1) Day 1: Frozen human peripheral blood mononuclear cells (PBMCs) were thawed, resuspended, and counted. 1×10⁶ PBMCs were added to each well of 24-well plates not treated for tissue culture, and complete medium (X-VIVO 15 serum-free medium, 2% FBS (inactivated by heating at 56°C for 30 minutes), referred to as complete medium hereinafter) in which IL-15 (5 ng/mL) and IL-7 (10 ng/mL) were added was supplemented to a final volume of 2 mL.
(2) Day 2: Before infection, the target cells were collected and counted. A specific number of cells was collected into sterile 1.5 mL Eppendorf tubes (approximately 5×10⁵ cells per infection reaction), and centrifuged at 500 g for 3 minutes at 4°C. The medium was discarded.
(2) The HLA-VSV-G bispecific molecules were diluted with pre-chilled complete medium to achieve final concentrations of 1.25 and 0.00 µg/mL, respectively (meanwhile, 2.5 µg/mL of antibodies targeting only HLA (without the LDLR-CR2-CR3 domain) was used as a control). The centrifuged cells from step (1) were resuspended with 50 µL of the mixture and incubated in a 4°C refrigerator for 15 minutes. After incubation, 1 mL of pre-chilled complete medium was added to the cell suspension to dilute the concentration of bispecific molecules in the system. The mixture was centrifuged at 500 g for 3 minutes at 4°C. The medium was discarded.
(3) The cells were resuspended again with 1 mL of pre-chilled complete medium and centrifuged at 500 g for 3 minutes at 4°C. The medium was discarded. This step was repeated once more.
(4) The mixture of VSV-G pseudotyped lentiviruses for infection was prepared. The viruses were diluted to an MOI of approximately 1 using pre-chilled complete medium. The cells collected by centrifugation in step (3) (with bispecific molecules bound to their surface at this time) were resuspended with 0.3 mL of the virus mixture, supplemented with IL-15 (5 ng/mL) and IL-7 (10 ng/mL), gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 500 g for 3 minutes at room temperature. The medium was discarded. The cells were resuspended with 1 mL of complete medium pre-warmed to 37°C, supplemented with IL-15 (5 ng/mL) and IL-7 (10 ng/mL), and inoculated into 24-well plates. The cell status and density were checked daily. If necessary, the medium was replaced with fresh medium or the cell culture volume was expanded.
(6) Day 4: 72 hours after infection, the infection efficiency was detected using a flow cytometer, with uninfected cells used as a control. The results are shown in FIG. 10.

### Example 6: Enhancement of Lentiviral Infection of Ramos Cells Using Bispecific Molecules (Targeting CD19)

(1) Day 1: Target cells (Ramos cells) were cultured in IMDM medium (the medium was supplemented with 10% FBS (inactivated by heating at 56°C for 30 minutes), 1% penicillin-streptomycin, and 1% GlutaMAX, referred to as IMDM complete medium hereinafter). Before infection, the target cells were collected and counted. A specific number of cells was collected into sterile 1.5 mL Eppendorf tubes (approximately 5×10⁵ cells per infection reaction) and centrifuged at 300 g for 3 minutes at 4°C. The medium was discarded.
(2) The CD19scfv-VSV-G bispecific molecules were diluted with pre-chilled IMDM medium (supplemented with 2% FBS) to achieve final concentration of 5 or 0 µg/mL. The centrifuged cells from step (1) were resuspended with 50 µL of the mixture and incubated in a 4°C refrigerator for 15 minutes. After incubation, 1 mL of pre-chilled IMDM medium (supplemented with 2% FBS) was added to the cell suspension to dilute the concentration of bispecific molecules in the system. The mixture was centrifuged at 300 g for 3 minutes at 4°C. The medium was discarded.
(3) The cells were resuspended again with 1 mL of pre-chilled IMDM medium (supplemented with 2% FBS) and centrifuged at 300 g for 3 minutes at 4°C. The medium was discarded. This step was repeated once more.
(4) The mixture of VSV-G pseudotyped lentiviruses for infection was prepared. The viruses were diluted to an MOI of approximately 1 using pre-chilled IMDM complete medium. The cells collected by centrifugation in step (3) (with bispecific molecules bound to their surface at this time) were resuspended with 0.5 mL of the virus mixture, gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 300 g for 3 minutes at room temperature. The medium was discarded. The cells were resuspended with 1 mL of IMDM complete medium pre-warmed to 37°C and inoculated into 24-well plates. The cell status and density were checked daily. If necessary, the medium was replaced with fresh medium or the cell culture volume was expanded to ensure that the cell density did not exceed 3×10⁶ cells/mL.
(6) Day 4: 72 hours after infection, the infection efficiency was detected using a flow cytometer, with uninfected cells used as a control. The results are shown in FIG. 11.

### Example 7: Enhancement of Lentiviral Infection of Ramos Cells Using Bispecific Molecules (Targeting CD20)

(1) Day 1: Target cells (Ramos cells) were cultured in IMDM medium (the medium was supplemented with 10% FBS (inactivated by heating at 56°C for 30 minutes), 1% penicillin-streptomycin, and 1% GlutaMAX, referred to as IMDM complete medium hereinafter). Before infection, the target cells were collected and counted. A specific number of cells was collected into sterile 1.5 mL Eppendorf tubes (approximately 5×10⁵ cells per infection reaction) and centrifuged at 300 g for 3 minutes at 4°C. The medium was discarded.
(2) The CD20-VSV-G bispecific molecules were diluted with pre-chilled IMDM medium (supplemented with 2% FBS) to achieve final concentration of 5 or 0 µg/mL. The centrifuged cells from step (1) were resuspended with 50 µL of the mixture and incubated in a 4°C refrigerator for 15 minutes. After incubation, 1 mL of pre-chilled IMDM medium (supplemented with 2% FBS) was added to the cell suspension to dilute the concentration of bispecific molecules in the system. The mixture was centrifuged at 300 g for 3 minutes at 4°C. The medium was discarded.
(3) The cells were resuspended again with 1 mL of pre-chilled IMDM medium (supplemented with 2% FBS) and centrifuged at 300 g for 3 minutes at 4°C. The medium was discarded. This step was repeated once more.
(4) The mixture of VSV-G pseudotyped lentiviruses for infection was prepared. The viruses were diluted to an MOI of approximately 1 using pre-chilled IMDM complete medium. The cells collected by centrifugation in step (3) (with bispecific molecules bound to their surface at this time) were resuspended with 0.5 mL of the virus mixture, gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 300 g for 3 minutes at room temperature. The medium was discarded. The cells were resuspended with 1 mL of IMDM complete medium pre-warmed to 37°C and inoculated into 24-well plates. The cell status and density were checked daily. If necessary, the medium was replaced with fresh medium or the cell culture volume was expanded to ensure that the cell density did not exceed 3×10⁶ cells/mL.
(6) Day 4: 60 hours after infection, the infection efficiency was detected using a flow cytometer. The results are shown in FIG. 12.

### Example 8: Enhancement of Lentiviral Infection of Ramos Cells Using Bispecific Molecules (Targeting CD45)

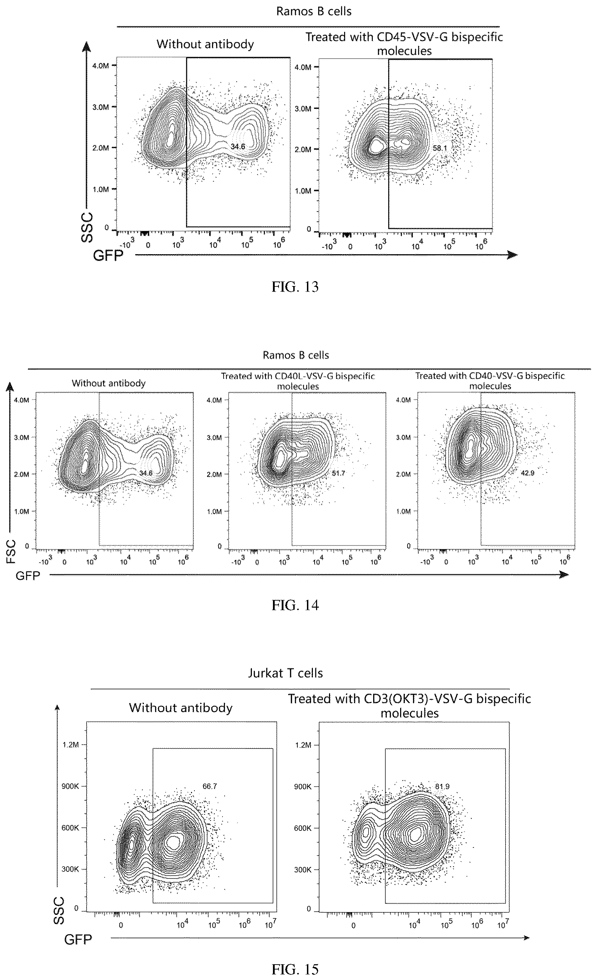

(1) Day 1: Target cells (Ramos cells) were cultured in IMDM medium (the medium was supplemented with 10% FBS (inactivated by heating at 56°C for 30 minutes), 1% penicillin-streptomycin, 1% GlutaMAX, referred to as IMDM complete medium hereinafter). Before infection, the target cells were collected and counted. A specific number of cells was collected into sterile 1.5 mL Eppendorf tubes (approximately 5×10⁵ cells per infection reaction) and centrifuged at 300 g for 3 minutes at 4°C. The medium was discarded.
(2) The CD40-VSV-G bispecific molecules were diluted with pre-chilled IMDM medium (supplemented with 2% FBS) to achieve final concentration of 5 or 0 µg/mL. The centrifuged cells from step (1) were resuspended with 50 µL of the mixture and incubated in a 4°C refrigerator for 15 minutes. After incubation, 1 mL of pre-chilled IMDM medium (supplemented with 2% FBS) was added to the cell suspension to dilute the concentration of bispecific molecules in the system. The mixture was centrifuged at 300 g for 3 minutes at 4°C. The medium was discarded.
(3) The cells were resuspended again with 1 mL of pre-chilled IMDM medium (supplemented with 2% FBS) and centrifuged at 300 g for 3 minutes at 4°C. The medium was discarded. This step was repeated once more.
(4) The mixture of VSV-G pseudotyped lentiviruses for infection was prepared. The viruses were diluted to an MOI of approximately 1 using pre-chilled IMDM complete medium. The cells collected by centrifugation in step (3) (with bispecific molecules bound to their surface at this time) were resuspended with 0.5 mL of the virus mixture, gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 300 g for 3 minutes at room temperature. The medium was discarded. The cells were resuspended with 1 mL of IMDM complete medium pre-warmed to 37°C and inoculated into 24-well plates. The cell status and density were checked daily. If necessary, the medium was replaced with fresh medium or the cell culture volume was expanded to ensure that the cell density did not exceed 3×10⁶ cells/mL.
(6) Day 4: 72 hours after infection, the infection efficiency was detected using a flow cytometer. The results are shown in FIG. 13.

### Example 9: Enhancement of Lentiviral Infection of Ramos Cells Using Bispecific Molecules (Targeting CD40)

(1) Day 1: Target cells (Ramos cells) were cultured in IMDM medium (the medium was supplemented with 10% FBS (inactivated by heating at 56°C for 30 minutes), 1% penicillin-streptomycin, 1% GlutaMAX, referred to as IMDM complete medium hereinafter). Before infection, the target cells were collected and counted. A specific number of cells was collected into sterile 1.5 mL Eppendorf tubes (approximately 5×10⁵ cells per infection reaction) and centrifuged at 300 g for 3 minutes at 4°C. The medium was discarded.
(2) The CD40-VSV-G or CD40L-VSV-G bispecific molecules were diluted with pre-chilled IMDM medium (supplemented with 2% FBS) to achieve final concentration of 5 µg/mL (meanwhile, the antibody-free treatment was used as a control). The centrifuged cells from step (1) were resuspended with 50 µL of the mixture and incubated in a 4°C refrigerator for 15 minutes. After incubation, 1 mL of pre-chilled IMDM medium (supplemented with 2% FBS) was added to the cell suspension to dilute the concentration of bispecific molecules in the system. The mixture was centrifuged at 300 g for 3 minutes at 4°C. The medium was discarded.
(3) The cells were resuspended again with 1 mL of pre-chilled IMDM medium (supplemented with 2% FBS) and centrifuged at 300 g for 3 minutes at 4°C. The medium was discarded. This step was repeated once more.
(4) The mixture of VSV-G pseudotyped lentiviruses for infection was prepared. The viruses were diluted to an MOI of approximately 1 using pre-chilled IMDM complete medium. The cells collected by centrifugation in step (3) (with bispecific molecules bound to their surface at this time) were resuspended with 0.5 mL of the virus mixture, gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 300 g for 3 minutes at room temperature. The medium was discarded. The cells were resuspended with 1 mL of IMDM complete medium pre-warmed to 37°C and inoculated into 24-well plates. The cell status and density were checked daily. If necessary, the medium was replaced with fresh medium or the cell culture volume was expanded to ensure that the cell density did not exceed 3×10⁶ cells/mL.
(6) Day 4: 60 hours after infection, the infection efficiency was detected using a flow cytometer. The results are shown in FIG. 14.

### Example 10: Enhancement of Lentiviral Infection of Jurkat T Cells Using Bispecific Molecules (Targeting CD3)

(1) Day 1: Target cells (Jurkat T cells) were cultured in RPMI-1640 medium (the medium was supplemented with 10% FBS (inactivated by heating at 56°C for 30 minutes), 1% penicillin-streptomycin, 1% GlutaMAX, 0.1% 2-mercaptoethanol (55 mM), referred to as RPMI-1640 complete medium hereinafter). Before infection, the target cells were collected and counted. A specific number of cells was collected into sterile 1.5 mL Eppendorf tubes (approximately 5×10⁵ cells per infection reaction), and centrifuged at 300 g for 3 minutes at 4°C. The medium was discarded.
(2) The CD3(OKT3)-VSV-G bispecific molecules were diluted with pre-chilled RPMI-1640 medium (supplemented with 2% FBS) to achieve final concentration of 5 or 0 µg/mL. The centrifuged cells from step (1) were resuspended with 50 µL of the mixture and incubated in a 4°C refrigerator for 15 minutes. After incubation, 1 mL of pre-chilled RPMI-1640 medium (supplemented with 2% FBS) was added to the cell suspension to dilute the concentration of bispecific molecules in the system. The mixture was centrifuged at 300 g for 3 minutes at 4°C. The medium was discarded.
(3) The cells were resuspended again with 1 mL of pre-chilled RPMI-1640 medium (supplemented with 2% FBS) and centrifuged at 300 g for 3 minutes at 4°C. The medium was discarded. This step was repeated once more.
(4) The mixture of VSV-G pseudotyped lentiviruses for infection was prepared. The viruses were diluted to an MOI of approximately 2 using pre-chilled RPMI-1640 complete medium. The cells collected by centrifugation in step (3) (with bispecific molecules bound to their surface at this time) were resuspended with 0.5 mL of the virus mixture, gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 300 g for 3 minutes at room temperature. The medium was discarded. The cells were resuspended with 1 mL of RPMI-1640 complete medium pre-warmed to 37°C and inoculated into 24-well plates. The cell status and density were checked daily. If necessary, the medium was replaced with fresh medium or the cell culture volume was expanded to ensure that the cell density did not exceed 3×10⁶ cells/mL.
(6) Day 4: 60 hours after infection, the infection efficiency was detected using a flow cytometer. The results are shown in FIG. 15.

### Example 11: Enhancement of Lentiviral Infection of Unstimulated Human Primary T Cells Using Bispecific Molecules (Targeting CD3)

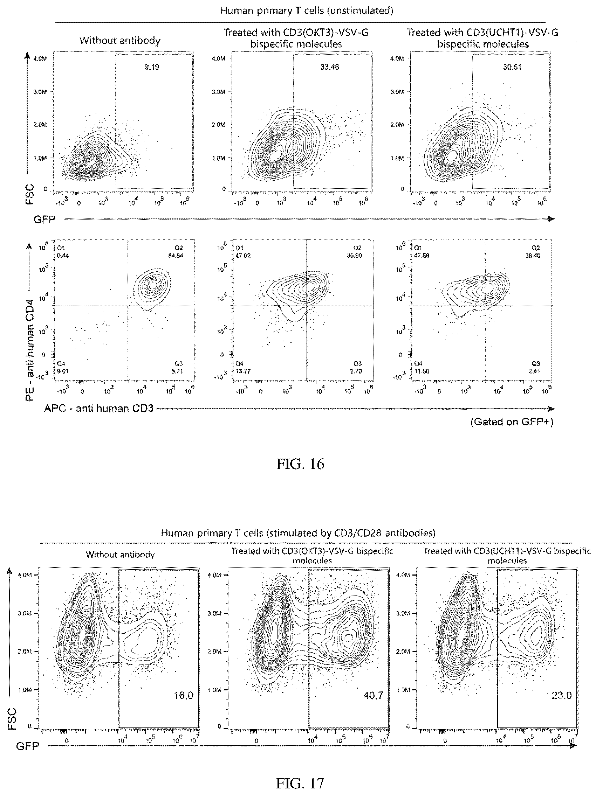

(1) Day 1: Frozen human peripheral blood mononuclear cells (PBMCs) were thawed, resuspended, and counted. 1×10⁶ PBMCs were added to each well of 24-well plates not treated for tissue culture, and complete medium (X-VIVO 15 serum-free medium, 2% FBS (inactivated by heating at 56°C for 30 minutes), referred to as complete medium hereinafter) in which IL-15 (5 ng/mL) and IL-7 (10 ng/mL) were added was supplemented to a final volume of 2 mL.
(2) Day 2: Before infection, the target cells were collected and counted. A specific number of cells was collected into sterile 1.5 mL Eppendorf tubes (approximately 5×10⁵ cells per infection reaction), and centrifuged at 500 g for 3 minutes at 4°C. The medium was discarded.
(2) The CD3(OKT)-VSV-G or CD3(UCHT1)-VSV-G bispecific molecules were diluted with pre-chilled complete medium to achieve a final concentration of 5 µg/mL (meanwhile, the antibody-free treatment was used as a control). The centrifuged cells from step (1) were resuspended with 50 µL of the mixture and incubated in a 4°C refrigerator for 15 minutes. After incubation, 1 mL of pre-chilled complete medium was added to the cell suspension to dilute the concentration of bispecific molecules in the system. The mixture was centrifuged at 500 g for 3 minutes at 4°C. The medium was discarded.
(3) The cells were resuspended again with 1 mL of pre-chilled complete medium and centrifuged at 500 g for 3 minutes at 4°C. The medium was discarded. This step was repeated once more.
(4) The mixture of VSV-G pseudotyped lentiviruses for infection was prepared. The viruses were diluted to an MOI of approximately 5 using pre-chilled complete medium. The cells collected by centrifugation in step (3) (with bispecific molecules bound to their surface at this time) were resuspended with 0.5 mL of the virus mixture, supplemented with IL-15 (5 ng/mL) and IL-7 (10 ng/mL), gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 500 g for 3 minutes at room temperature. The medium was discarded. The cells were resuspended with 1 mL of complete medium pre-warmed to 37°C, supplemented with IL-15 (5 ng/mL) and IL-7 (10 ng/mL), and inoculated into 24-well plates. The cell status and density were checked daily. If necessary, the medium was replaced with fresh medium or the cell culture volume was expanded.
(6) Day 4: 72 hours after infection, the infection efficiency was detected using a flow cytometer. The results are shown in FIG. 16.

### Example 12: Enhancement of Lentiviral Infection of Human Primary T Cells Stimulated by CD3/CD28 Using Bispecific Molecules (Targeting CD3)

(1) Day 1: CD3 and CD28 antibodies were diluted in sterile water to a final concentration of 1 µg/mL. 0.5 mL (= 0.5 µg of antibodies) of the antibody solution was added to each well of 24-well plates not treated for tissue culture by a pipette, and incubated in an incubator at 37°C for 4 hours. The antibody solution was aspirated, and 1 mL of complete medium (X-VIVO 15 serum-free medium, 2% FBS (inactivated by heating at 56°C for 30 minutes), referred to as complete medium hereinafter) was added to each well, followed by incubation in the incubator at 37°C for 15-30 minutes. During the period, forzen PBMCs were thawed, resuspended, and counted. The medium was aspirated. 1×10⁶ PBMC cells were added to each well, and complete medium was supplemented to a final volume of 2 mL. PBS could be added to empty wells to prevent medium evaporation.
(2) Day 2: IL-15 (5 ng/mL) and IL-7 (10 ng/mL) were added.
(3) Day 4: Before infection, the target cells were collected and counted. A specific number of cells was collected into sterile 1.5 mL Eppendorf tubes (approximately 5×10⁵ cells per infection reaction), and centrifuged at 300 g for 3 minutes at 4°C. The medium was discarded.
(2) The CD3(OKT)-VSV-G or CD3(UCHT1)-VSV-G bispecific molecules were diluted with pre-chilled complete medium to achieve a final concentration of 5 µg/mL (meanwhile, the antibody-free treatment was used as a control). The centrifuged cells from step (1) were resuspended with 50 µL of the mixture and incubated in a 4°C refrigerator for 15 minutes. After incubation, 1 mL of pre-chilled complete medium was added to the cell suspension to dilute the concentration of bispecific molecules in the system. The mixture was centrifuged at 500 g for 3 minutes at 4°C. The medium was discarded.
(3) The cells were resuspended again with 1 mL of pre-chilled complete medium and centrifuged at 500 g for 3 minutes at 4°C. The medium was discarded. This step was repeated once more.
(4) The mixture of VSV-G pseudotyped lentiviruses for infection was prepared. The viruses were diluted to an MOI of approximately 5 using pre-chilled complete medium. The cells collected by centrifugation in step (3) (with bispecific molecules bound to their surface at this time) were resuspended with 0.5 mL of the virus mixture, supplemented with IL-15 (5 ng/mL) and IL-7 (10 ng/mL), gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 500 g for 3 minutes at room temperature. The medium was discarded. The cells were resuspended with 1 mL of complete medium pre-warmed to 37°C, supplemented with IL-15 (5 ng/mL) and IL-7 (10 ng/mL), and inoculated into 24-well plates. The cell status and density were checked daily. If necessary, the medium was replaced with fresh medium or the cell culture volume was expanded.
(6) Day 4: 72 hours after infection, the infection efficiency was detected using a flow cytometer. The results are shown in FIG. 17.

### Example 13: Enhancement of Lentiviral Infection of Unstimulated Human Primary T Cells Using Bispecific Molecules (Targeting CD4)

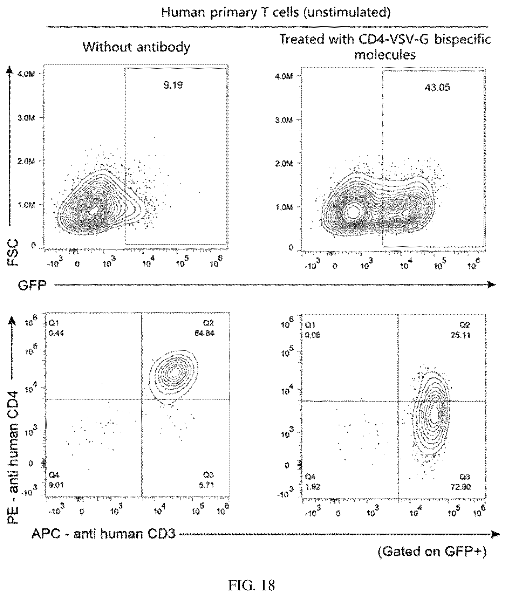

(1) Day 1: Frozen human peripheral blood mononuclear cells (PBMCs) were thawed, resuspended, and counted. 1×10⁶ PBMCs were added to each well of a 24-well plate not treated for tissue culture, and complete medium (X-VIVO 15 serum-free medium, 2% FBS (inactivated by heating at 56°C for 30 minutes), referred to as complete medium hereinafter) in which IL-15 (5 ng/mL) and IL-7 (10 ng/mL) were added was supplemented to a final volume of 2 mL.
(2) Day 2: Before infection, the target cells were collected and counted. A specific number of cells was collected into sterile 1.5 mL Eppendorf tubes (approximately 5×10⁵ cells per infection reaction), and centrifuged at 500 g for 3 minutes at 4°C. The medium was discarded.
(2) The CD4 bispecific molecules were diluted with pre-chilled complete medium to achieve a final concentration of 5 µg/mL (meanwhile, the antibody-free treatment was used as a control). The centrifuged cells from step (1) were resuspended with 50 µL of the mixture and incubated in a 4°C refrigerator for 15 minutes. After incubation, 1 mL of pre-chilled complete medium was added to the cell suspension to dilute the concentration of bispecific molecules in the system. The mixture was centrifuged at 500 g for 3 minutes at 4°C. The medium was discarded.
(3) The cells were resuspended again with 1 mL of pre-chilled complete medium and centrifuged at 500 g for 3 minutes at 4°C. The medium was discarded. This step was repeated once more.
(4) The mixture of VSV-G pseudotyped lentiviruses for infection was prepared. The viruses were diluted to an MOI of approximately 5 using pre-chilled complete medium. The cells collected by centrifugation in step (3) (with bispecific molecules bound to their surface at this time) were resuspended with 0.5 mL of the virus mixture, supplemented with IL-15 (5 ng/mL) and IL-7 (10 ng/mL), gently pipetted to mix evenly, and then inoculated into 24-well plates. They were centrifuged at 1800 g for 45 minutes at 37°C (acceleration 9, deceleration 5). After centrifugation, the cells were placed in a 37°C incubator with 5% CO₂ for culture.
(5) Day 2: 24 hours after infection, the cells were gently pipetted and all transferred to sterile 1.5 mL Eppendorf tubes and centrifuged at 500 g for 3 minutes at room temperature. The medium was discarded. The cells were resuspended with 1 mL of complete medium pre-warmed to 37°C, supplemented with IL-15 (5 ng/mL) and IL-7 (10 ng/mL), and inoculated into 24-well plates. The cell status and density were checked daily. If necessary, the medium was replaced with fresh medium or the cell culture volume was expanded.
(6) Day 4: 72 hours after infection, the infection efficiency was detected using a flow cytometer. The results are shown in FIG. 18.

As can be seen from FIGS. 8 to 18, when infecting Jurkat T cells, Ramos cells and human primary T cells (unstimulated or stimulated with CD3/CD28 antibodies) with the same amount of VSV-G pseudotyped lentiviruses, the use of corresponding bispecific antibodies all can increase the infection efficiency of VSV-G pseudotyped lentiviruses. Among them, the use of HLA-VSV-G bispecific antibodies increased the infection efficiency of Ramos cells from 4.5% to a maximum of 53.8% (FIG. 8); the use of HLA-VSV-G bispecific antibodies increased the infection efficiency of Jurkat T cells from 38.0% to a maximum of 75.7% (FIG. 9); the use of HLA-VSV-G bispecific antibodies increased the infection efficiency of human peripheral blood mononuclear cells from 0.052% to 62.7% (FIG. 10); the use of CD29scFv-VSV-G bispecific antibodies increased the infection efficiency of Ramos cells from 21.4% to 59.4% (FIG. 11); the use of CD20-VSV-G bispecific antibodies increased the infection efficiency of Ramos cells from 34.6% to 68.2% (FIG. 12); the use of CD45-VSV-G bispecific antibodies increased the infection efficiency of Ramos cells from 34.6% to 58.1% (FIG. 13); the use of CD40-VSV-G or CD40L-VSV-G bispecific antibodies increased the infection efficiency of Ramos cells from 34.6% to 42.9% or 51.7% (FIG. 14); the use of CD3-VSV-G bispecific antibodies increased the infection efficiency of Jurkat T cells from 66.7% to a maximum of 81.9% (FIG. 15); the use of CD3(OKT3)-VSV-G or CD3(UCHT1)-VSV-G bispecific antibodies increased the infection efficiency of unstimulated human primary T cells from 9.19% to 33.46% or 30.61% (FIG. 16); the use of CD3(OKT3)-VSV-G or CD3(UCHT1)-VSV-G bispecific antibodies increased the infection efficiency of human primary T cells stimulated by CD3/CD28 antibodies from 16.0% to 40.7% or 23.0% (FIG. 17); the use of CD4-VSV-G bispecific antibodies increased the infection efficiency of unstimulated human primary T cells from 9.19% to 43.05% (FIG. 18). Meanwhile, by comparing the staining of specific proteins on the surface of T cells in FIG. 18 and FIG. 16, it can be seen that after lentiviral infection mediated by CD3-targeting bispecific molecules, the expression of CD3 on the cell surface was down-regulated; lentiviral infection mediated by CD4-targeting bispecific molecules led to the down-regulation of CD4 expression on the cell surface, which further indicates that lentiviruses enter cells through the action of bispecific molecules.

In addition, according to the analysis of the mean fluorescence intensity (MFI) results in FIGS. 8 to 18, it is found that the bispecific antibodies of the present disclosure not only enhanced the viral infection efficiency but also increased the expression level of the target gene (GFP) in cells. Among them, the use of HLA-VSV-G bispecific antibodies increased the expression level of the target proteins in Ramos cells from 49038 to a maximum of 165640 (FIG. 8); the use of HLA-VSV-G bispecific antibodies increased the expression level of the target proteins in Jurkat T cells from 8689 to a maximum of 17143 (FIG. 9); the use of HLA-VSV-G bispecific antibodies increased the expression level of the target proteins in human peripheral blood mononuclear cells from 227 to 4489 (FIG. 10); the use of CD29scFv-VSV-G bispecific antibodies increased the expression level of the target proteins in Ramos cells from 5238 to 11043 (FIG. 11); the use of CD20-VSV-G bispecific antibodies increased the expression level of the target proteins in Ramos cells from 127289 to 163324 (FIG. 12); the use of CD3-VSV-G bispecific antibodies increased the expression level of the target proteins in Jurkat T cells from 18338 to 32697 (FIG 15); the use of CD3(OKT3)-VSV-G or CD3(UCHT1)-VSV-G bispecific antibodies increased the expression level of the target proteins in unstimulated human primary T cells from 10264 to 58730 or 34160 (FIG. 16); the use of CD3(OKT3)-VSV-G or CD3(UCHT1)-VSV-G bispecific antibodies increased the expression level of the target proteins in human primary T cells stimulated by CD3/CD28 antibodies from 225907 to 453000 or 337512 (FIG. 17); the use of CD4-VSV-G bispecific antibodies increased the expression level of the target proteins in unstimulated human primary T cells from 10264 to 22693 (FIG. 18).

It demonstrates that the bispecific antibodies of the present disclosure can improve the infection efficiency and expression efficiency of VSV-G pseudotyped lentiviruses in different cells.

## Claims

1. A bispecific antigen-binding molecule or a fragment thereof, comprising a first antigen-binding domain and a second antigen-binding domain, wherein the first antigen-binding domain is used for specifically binding to a cell surface antigen; the second antigen-binding domain is used for specifically binding to a vesicular stomatitis virus glycoprotein (VSV-G).

2. The bispecific antigen-binding molecule or the fragment thereof according to claim 1, wherein the first antigen-binding domain comprises a heavy chain (HC) variable region (VH) and/or a light chain (LC) variable region (VL).

3. The bispecific antigen-binding molecule or the fragment thereof according to claim 1, wherein the first antigen-binding domain further comprises a heavy chain (HC) constant region (CH) or a fragment thereof and/or a light chain (LC) constant region (CL) or a fragment thereof; preferably, the heavy chain constant region or the fragment thereof is an IgG constant region; and/or the light chain constant region is a κ constant region or a λ constant region.

4. The bispecific antigen-binding molecule or the fragment thereof according to claim 1, wherein the first antigen-binding domain comprises at least one of Fab, Fab', F(ab')2, scFv, a natural ligand of a cell surface protein, or VHH.

5. The bispecific antigen-binding molecule or the fragment thereof according to claim 1, wherein the first antigen-binding domain is a monoclonal antibody, a humanized antibody, a human antibody, a chimeric antibody or an affinity-optimized antibody.

6. The bispecific antigen-binding molecule or the fragment thereof according to claim 1, wherein the first antigen-binding domain further comprises an Fc domain; preferably, the Fc domain comprises at least one mutation capable of reducing or enhancing the ADCC activity of the bispecific antibody.

7. The bispecific antigen-binding molecule or the fragment thereof according to claim 1, wherein the cell surface antigen is selected from a B cell surface antigen, an NK cell surface antigen, a hematopoietic stem cell surface antigen or a T cell surface antigen; preferably, the cell surface antigen is selected from any one of CD3, CD19, CD45, CD20, CD34, CD40, CD56, CD16, CD133, CD147, CD123, CD138, CD22, CD30, CD33, CD38, CD70, CD4, CD5, CD8A & CD8B, CD7 and HLA.

8. The bispecific antigen-binding molecule or the fragment thereof according to claim 1, wherein the second antigen-binding domain comprises CR2 and CR3 domains of a low-density lipoprotein receptor (LDL-R).

9. The bispecific antigen-binding molecule or the fragment thereof according to claim 8, wherein the second antigen-binding domain comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.1.

10. The bispecific antigen-binding molecule or the fragment thereof according to claim 1, wherein
(a) the second antigen-binding domain is covalently linked to the carboxyl terminus of the heavy chain of the first antigen-binding domain;
(b) the second antigen-binding domain is covalently linked to the amino terminus of the heavy chain of the first antigen-binding domain; or
(c) the second antigen-binding domain is covalently embedded in the polypeptide chain of the heavy chain of the first antigen-binding domain;
and/or
(d) the second antigen-binding domain is covalently linked to the carboxyl terminus of the light chain of the first antigen-binding domain;
(e) the second antigen-binding domain is covalently linked to the amino terminus of the light chain of the first antigen-binding domain; or
(f) the second antigen-binding domain is covalently embedded in the polypeptide chain of the light chain of the first antigen-binding domain.

11. The bispecific antigen-binding molecule or the fragment thereof according to claim 1, wherein the second antigen-binding domain is linked to the heavy chain and/or light chain of the first antigen-binding domain via a peptide linker; preferably, the peptide linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

12. A bispecific antigen-binding molecule or a fragment thereof, comprising:
a first polypeptide comprising a VH, a CH1, a CH2, a CH3, a CR2 and a CR3; and a second polypeptide comprising a VL and a CL;
wherein VH is the heavy chain variable region of the first antigen-binding domain, VL is the light chain variable region of the first antigen-binding domain, the first antigen-binding domain is used for specifically binding to a cell surface antigen; CH1, CH2 and CH3 are respectively the first, second, and third constant regions of an IgG molecule, CL is the light chain constant region; CR2 and CR3 are the second and third CR domains of a low-density lipoprotein receptor (LDL-R).

13. The bispecific antigen-binding molecule or the fragment thereof according to claim 12, wherein the first polypeptide comprises VH, CH1, CH2, CH3, CR2 and CR3 in the order from the N-terminus to the C-terminus; the second polypeptide comprises VL and CL in the order from the N-terminus to the C-terminus;
preferably, the CR2 and CR3 domains comprise an amino acid sequence that is at least 90%, at least 91%, at least 92%, at least 93%, at least 94%, at least 95%, at least 96%, at least 97%, at least 98%, at least 99%, or 100% sequence identical to the sequence shown in SEQ ID NO.1.

14. The bispecific antigen-binding molecule or the fragment thereof according to claim 12, wherein the two polypeptides associate to form an antigen-binding site targeting a cell surface antigen; preferably, the cell surface antigen is selected from a B cell surface antigen, an NK cell surface antigen, a hematopoietic stem cell surface antigen or a T cell surface antigen; preferably, the cell surface antigen is selected from any one of CD3, CD19, CD45, CD20, CD34, CD40, CD56, CD16, CD133, CD147, CD123, CD138, CD22, CD30, CD33, CD38, CD70, CD4, CD5, CD8A & CD8B, CD7 and HLA.

15. The bispecific antigen-binding molecule or the fragment thereof according to claim 12, wherein the bispecific antigen-binding molecule is tetravalent or hexavalent; preferably, the bispecific antigen-binding molecule is bivalent or tetravalent for two targets.

16. The bispecific antigen-binding molecule or the fragment thereof according to claim 13, wherein the first polypeptide comprises a peptide linker between CH3 and CR2; preferably, the peptide linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO. 10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

17. The bispecific antigen-binding molecule or the fragment thereof according to claim 12, wherein the first polypeptide comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.2; the second polypeptide comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.3; or
the first polypeptide comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.4; the second polypeptide comprises an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.5.

18. The bispecific antigen-binding molecule or the fragment thereof according to claim 13, wherein the second polypeptide further comprises CR2 and CR3 domains in the order from the N-terminus to the C-terminus; preferably, the second polypeptide further comprises a peptide linker between CL and CR2; preferably, the peptide linker is selected from L1, L2, L3 or L4; wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

19. A bispecific antigen-binding molecule or a fragment thereof, comprising:
a first antigen-binding domain comprising an scFv, the scFv comprises a VH and a VL; and a second antigen-binding domain comprising a CR2 and a CR3; and optionally, CH2 and CH3 domains for linking the scFv and the second antigen-binding domain;
wherein the first antigen-binding domain is used for specifically binding to a cell surface antigen; VH is the heavy chain variable region, VL is the light chain variable region; CH2 and CH3 are respectively the second and third constant regions of an IgG molecule; CR2 and CR3 are the second and third CR domains of a low-density lipoprotein receptor (LDL-R).

20. The bispecific antigen-binding molecule or the fragment thereof according to claim 19, comprising scFv, CH2, CH3, CR2 and CR3 in the order from the N-terminus to the C-terminus; the scFv comprises VH and VL;
preferably, the CR2 and CR3 domains comprise an amino acid sequence that is at least 90%, at least 91%, at least 92%, at least 93%, at least 94%, at least 95%, at least 96%, at least 97%, at least 98%, at least 99%, or 100% sequence identical to the sequence shown in SEQ ID NO.1.

21. The bispecific antigen-binding molecule or the fragment thereof according to claim 20, wherein the scFv targets a cell surface antigen; preferably, the cell surface antigen is selected from a B cell surface antigen, an NK cell surface antigen, a hematopoietic stem cell surface antigen or a T cell surface antigen; preferably, the cell surface antigen is selected from any one of CD3, CD19, CD45, CD20, CD34, CD40, CD56, CD16, CD133, CD147, CD123, CD138, CD22, CD30, CD33, CD38, CD70, CD4, CD5, CD8A & CD8B, CD7 and HLA.

22. The bispecific antigen-binding molecule or the fragment thereof according to any one of claims 19 to 21, wherein the bispecific antigen-binding molecule is tetravalent.

23. The bispecific antigen-binding molecule or the fragment thereof according to any one of claims 19 to 21, wherein the bispecific antigen-binding molecule is bivalent for two targets.

24. The bispecific antigen-binding molecule or the fragment thereof according to claim 20, comprising a peptide linker between scFv and CH2; preferably, the peptide linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

25. The bispecific antigen-binding molecule or the fragment thereof according to claim 20, comprising a peptide linker between CH3 and CR2; preferably, the peptide linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

26. The bispecific antigen-binding molecule or the fragment thereof according to claim 19, comprising an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.6.

27. A bispecific antigen-binding molecule or a fragment thereof, comprising:
a first antigen-binding domain comprising a VHH; and a second antigen-binding domain comprising a CR2 and a CR3; and optionally, CH2 and CH3 domains for linking the VHH and the second antigen-binding domain;
wherein the first antigen-binding domain is for specifically binding to a cell surface antigen; the CH2 and CH3 are respectively the second and third constant regions of an IgG molecule; CR2 and
CR3 are the second and third CR domains of a low-density lipoprotein receptor (LDL-R).

28. The bispecific antigen-binding molecule or the fragment thereof according to claim 27, comprising VHH, CR2 and CR3 in the order from the N-terminus to the C-terminus; preferably, the CR2 and CR3 domains comprise an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.1.

29. The bispecific antigen-binding molecule or the fragment thereof according to claim 27, wherein the cell surface antigen is selected from a B cell surface antigen, an NK cell surface antigen, a hematopoietic stem cell surface antigen or a T cell surface antigen; preferably, the cell surface antigen is selected from any one of CD3, CD19, CD45, CD20, CD34, CD40, CD56, CD16, CD133, CD147, CD123, CD138, CD22, CD30, CD33, CD38, CD70, CD4, CD5, CD8A & CD8B, CD7 and HLA.

30. The bispecific antigen-binding molecule or the fragment thereof according to claim 27, wherein the bispecific antigen-binding molecule is bivalent; preferably, the bispecific antigen-binding molecule is monovalent for two targets.

31. The bispecific antigen-binding molecule or the fragment thereof according to claim 28, comprising a peptide linker between VHH and CR2; preferably, the linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

32. A bispecific antigen-binding molecule or a fragment thereof, comprising:
a first antigen-binding domain comprising a natural ligand of a cell surface protein; and a second antigen-binding domain comprising a CR2 and a CR3; and optionally, CH2 and CH3 domains for linking the natural ligand and the second antigen-binding domain;
wherein the first antigen-binding domain is used for specifically binding to a cell surface antigen;
CH2 and CH3 are respectively the second and third constant regions of an IgG molecule; CR2 and
CR3 are the second and third CR domains of a low-density lipoprotein receptor (LDL-R).

33. The bispecific antigen-binding molecule or the fragment thereof according to claim 32, comprising CR2, CR3, CH2, CH3 and a natural ligand in the order from N-terminus to C-terminus; preferably, the CR2 and CR3 domains comprise an amino acid sequence that is at least 90%, at least 91%, at least 92%, at least 93%, at least 94%, at least 95%, at least 96%, at least 97%, at least 98%, at least 99% or 100% sequence identical to the sequence shown in SEQ ID NO.1.

34. The bispecific antigen-binding molecule or the fragment thereof according to claim 32, wherein the cell surface antigen is selected from a B cell surface antigen, an NK cell surface antigen, a hematopoietic stem cell surface antigen or a T cell surface antigen; preferably, the cell surface antigen is selected from any one of CD3, CD19, CD45, CD20, CD34, CD40, CD56, CD16, CD133, CD147, CD123, CD138, CD22, CD30, CD33, CD38, CD70, CD4, CD5, CD8A & CD8B, CD7 and HLA.

35. The bispecific antigen-binding molecule or the fragment thereof according to claim 32, wherein the bispecific antigen-binding molecule is tetravalent; preferably, the bispecific antigen-binding molecule is bivalent for two targets.

36. The bispecific antigen-binding molecule or the fragment thereof according to claim 33, comprising a peptide linker between CR3 and CH2; preferably, the linker is selected from L1, L2, L3 or L4, wherein the sequence of L1 is shown in SEQ ID NO.7, the sequence of L2 is shown in SEQ ID NO.8, the sequence of L3 is shown in SEQ ID NO.9, and the sequence of L4 is shown in SEQ ID NO.10; preferably, the L1 peptide linker is G₄S (SEQ ID NO.11) or (G₄S)₃ (SEQ ID NO.12); more preferably, the L2 peptide linker is (G₄S)₃A (SEQ ID NO.13) or (G₄S)₄A (SEQ ID NO.14).

37. Use of the bispecific antigen-binding molecule or the fragment thereof according to any one of claims 1-36 for mediating a gene transduction of a receptor-independent lentiviral vector; preferably, the subject of the gene transduction is a cell with low expression or no expression of low-density lipoprotein receptors; preferably, the subject of the gene transduction is selected from a T cell, a B cell, an NK cell or a hematopoietic stem cell; preferably, the T cell and the B cell are primary cells.

38. A polynucleotide encoding the bispecific antigen-binding molecule or the fragment thereof according to any one of claims 1-36; or, a recombinant vector comprising the polynucleotide; or, a host cell comprising the polynucleotide or the recombinant vector.

39. A method for producing the bispecific antigen-binding molecule or the fragment thereof according to any one of claims 1-36.

40. A method for transducing a target gene into a subject using a lentiviral vector, comprising administering the bispecific antigen-binding molecule or the fragment thereof according to any one of claims 1-36 to the subject.
